# EUROPEAN PATENT APPLICATION

(11) **EP 2 210 941 A1**
(43) Date of publication of application: **28.07.2010**
(21) Application number: 08842183.9
(22) Date of filing: 24.10.2008
(51) Int. Cl.: C12N 15/09, C12N 9/12, C12Q 1/68

(54) **ISOTHERMAL AMPLIFICATION METHOD AND DNA POLYMERASE USED IN THE SAME**

(30) Priority: 25.10.2007 JP 2007277496
(71) Applicant: Riken, Wako-shi, Saitama 351-0198 (JP); Kabushiki Kaisha Dnaform, Kanagawa 230-0046 (JP)
(72) Inventor: HAYASHIZAKI, Yoshide, Kanagawa 230-0045 (JP); ITOH, Masayoshi, Saitama 351-0198 (JP); LEZHAVA, Alexander, Kanagawa 230-0045 (JP); BENNO, Yoshimi, Saitama 351-0198 (JP); MITANI, Yasumasa, Kanagawa 230-0046 (JP); KANAMORI, Hajime, Kanagawa 230-0046 (JP)
(74) Representative: Behnisch, Werner
(86) International application number: PCT/JP2008/069371
(87) International publication number: WO 2009/054510

(57) **Abstract**

A DNA polymerase suitable for specific isothermal amplification methods and an isothermal amplification method using the DNA polymerase are provided. In the presence of a DNA polymerase including a protein described in the following item (a) or (b), an amplification reaction of a target nucleic acid sequence in a nucleic acid sample is carried out isothermally using a first primer shown in the following (X). By using the DNA polymerase, it becomes possible to carry out the amplification reaction using the primer within a shorter time than ever before.
(a) a protein having an amino acid sequence represented by SEQ ID NO. 23
(b) a protein having an amino acid sequence represented by SEQ ID NO. 25

(X) a primer that contains, in a 3' end portion, a sequence (Ac') that hybridizes to a sequence (A) of a 3' end portion of the target nucleic acid sequence and also contains, on a 5' side of the sequence (Ac'), a sequence (B') that hybridizes to a complementary sequence (Bc) to a sequence (B) present on a 5' side with respect to the sequence (A) in the target nucleic acid sequence

## Description

### Technical Field

The present invention relates to an isothermal amplification method and a DNA polymerase used in the same.

### Background Art

DNA polymerases are some of the most widely used enzymes in the field of life science, and they are essential in various techniques including a polymerase chain reaction (PCR) method, for example. Many kinds of such DNA polymerases are commercially available, and each polymerase is characterized by the reaction conditions therefor and an enzyme activity(s) thereof, for example. The most well known DNA polymerase I from *Escherichia coli* has a 5' → 3' polymerase activity, which allows, from a template DNA and a primer, a complementary sequence to the template to be synthesized, and also has a 5' → 3' exonuclease activity and a 3' → 5' exonuclease activity. It is known that these three enzyme activities pertain to different structural domains. That is, they pertain to a 5' → 3' exonuclease domain on an N-terminal side, a 3' → 5' exonuclease domain in a central portion, and a polymerase domain on a C-terminal side, respectively (see Non-Patent Document 1, for example). A large fragment of 75 kD obtained by treating the DNA polymerase I with subtilisin is also called "Klenow fragment", and it lacks the 5' → 3' exonuclease activity among the above-described three activities. Thus, the Klenow fragment is useful in a sequence reaction according to a dideoxy method, a reaction for blunting a 5'-protruding end, and the like. Currently, Klenow fragments expressed and purified as recombinant proteins of the DNA polymerase I with no small fragments on the N-terminal side are commercially available.

As a DNA amplification technique, a PCR method generally is used. However, the PCR method has problems in that complicated temperature control is required, a thermal cycler for conducting such complicated temperature control is required, it takes several hours to complete the reaction, etc. Thus, as a DNA amplification technique as an alternative to the PCR method, a LAMP (Loop-mediated Isothermal Amplification) method (see Non-Patent Document 2, for example), a SDA (Strand Displacement Amplification) method (see Patent Document 1, for example), a method proposed by Mitani et al. (see Patent Documents 4, 5, and 6, and Non-Patent Document 3, for example), and the like have been developed. These methods are called isothermal amplification methods because amplification reactions in these methods can be carried out isothermally. In these methods, complicated temperature control and a thermal cycler for conducting it as required in PCR are not necessary. On the other hand, a DNA polymerase having a complementary strand displacement replication activity (see Patent Documents 2 and 3, for example) is essential for the isothermal amplification reactions. Currently, only a few kinds of DNA polymerase having a complementary strand displacement replication activity are available on the market, and it has been pointed out that reaction conditions therefor such as an optimum temperature are limited, a reaction time is long, or the like. Such problems place restrictions on the development of test agents, diagnostic agents, etc. using these DNA amplification methods.
[Patent Document 1] JP 10(1998)-313900 A
[Patent Document 2] Japanese Patent No. 2978001
[Patent Document 3] JP 09(1997)-224681 A
[Patent Document 4] WO 2004/040019
[Patent Document 5] WO 2005/063977
[Patent Document 6] WO 2001/030993
[Non-Patent Document 1] Kornberg, A., Baker TA. DNA Replication, W. H. Freeman and Company, New York, 1992.
[Non-Patent Document 2] Notomi, T. et al., Nucleic Acids Research, 2000, Vol. 28, No. 12, e63
[Non-Patent Document 3] Mitani, Y. et al., Nature Methods, 2007, Vol. 4, No. 3, 257-262

### Disclosure of Invention

The DNA polymerases currently used in the isothermal amplification methods act at relatively low temperatures. Thus, in the amplification reactions, specificity in annealing between a template DNA and a primer is low. Accordingly, it has been pointed out that there are problems such that by-products are produced owing to the decreased specificity in the amplification reactions and that it is difficult to amplify a relatively long target sequence. Furthermore, even if the same DNA polymerase is used, for example, the amplification efficiency varies depending on the isothermal amplification method to which it is applied. Thus, DNA polymerases suitable for the respective isothermal amplification methods are demanded.

With the foregoing in mind, it is an object of the present invention to provide a DNA polymerase suitable for specific isothermal amplification methods and an isothermal amplification method using the same.

An isothermal amplification method of the present invention is an isothermal amplification method for carrying out isothermal amplification of a target nucleic acid sequence in a nucleic acid sample. The method includes carrying out an amplification reaction of the target nucleic acid sequence isothermally in the presence of a DNA polymerase composed of a protein described in any of the following items (a) to (d) using a first primer shown in the following (X):
(a) a protein having an amino acid sequence represented by SEQ ID NO. 23;
(b) a protein having an amino acid sequence represented by SEQ ID NO. 25;
(c) a protein having an amino acid sequence obtained by deletion of any number from 1 to 334 of consecutive amino acid residues starting from an N-terminal in the amino acid sequence represented by SEQ ID NO. 23;
(d) a protein having an amino acid sequence obtained by deletion, substitution, insertion, or addition of one or more amino acids in the amino acid sequence of the protein described in any of the items (a) to (c) and having a DNA polymerase activity; and
(X) a primer that contains, in a 3' end portion, a sequence (Ac') that hybridizes to a sequence (A) of a 3' end portion of the target nucleic acid sequence and also contains, on a 5' side of the sequence (Ac'), a sequence (B') that hybridizes to a complementary sequence (Bc) to a sequence (B) present on a 5' side with respect to the sequence (A) in the target nucleic acid sequence.

An isothermal amplification DNA polymerase according to the present invention is a DNA polymerase to be used in the isothermal amplification method of the present invention. The DNA polymerase is composed of a protein described in any of the following items (a) to (d).

An isothermal amplification kit of the present invention is an isothermal amplification kit to be used in the isothermal amplification method of the present invention. The kit includes the isothermal amplification DNA polymerase according to the present invention.

The inventors of the present invention found that thermostable *Alicyclobacillus acidocaldarius*-derived DNA polymerase (hereinafter referred to as "Aac polymerase") having a DNA replication activity and a complementary strand displacement replication activity is suitable for specific methods to be described later among various isothermal amplification methods, and thus achieved the present invention. The isothermal amplification DNA polymerase of the present invention can improve the amplification efficiencies of the specific isothermal amplification methods to be described later as compared with conventional DNA polymerases used in the isothermal amplification methods. As a result, the isothermal amplification DNA polymerase of the present invention allows nucleic acid amplification to be carried out in a shorter time than ever before. If the time required for nucleic acid amplification can be shortened, for example, analysis of single nucleotide polymorphism (SNP) or the like utilizing the nucleic acid amplification can be conducted more rapidly and a large amount of specimen can be analyzed more efficiently. Therefore, the present invention is very useful in the fields of nucleic acid analyses, clinical tests, and the like utilizing nucleic acid amplification. The fact that the Aac DNA polymerase is suitable for the specific isothermal amplification methods is newly discovered by the inventors of the present invention.

### Brief Description of Drawings

[FIG. 1] FIG. 1 is a graph showing a standard curve used in DNA polymerase activity measurement.
[FIG. 2] FIG. 2 is an electrophoretogram showing the result obtained when a complementary strand displacement replication activity was measured at 60°C to 68°C using the isothermal amplification DNA polymerase according to the present invention and a commercially available DNA polymerase.
[FIG. 3] FIG. 3 is an electrophoretogram showing the result obtained when the complementary strand displacement replication activity was measured at 68°C to 74°C using the isothermal amplification DNA polymerase according to the present invention and the commercially available DNA polymerase.
[FIG. 4] FIG. 4 is a graph showing an amplification profile obtained when an isothermal amplification reaction was carried out using the isothermal amplification DNA polymerase according to the present invention and a commercially available DNA polymerase.
[FIG. 5] FIG. 5 is a graph showing an amplification profile obtained when an isothermal amplification reaction was carried out using the isothermal amplification DNA polymerase according to the present invention and a commercially available DNA polymerase.
[FIG. 6] FIG. 6 is a graph showing an amplification profile obtained when an isothermal amplification reaction was carried out using the isothermal amplification DNA polymerase according to the present invention and a commercially available DNA polymerase.
[FIG. 7] FIG. 7 is a schematic diagram showing the mechanism of action of nucleic acid synthesis using a first primer in the SMAP method according to one embodiment of the present invention.
[FIG. 8] FIG. 8 is a schematic diagram showing an example of a second primer used in the SMAP method according to one embodiment of the present invention.
[FIG. 9] FIG. 9 is a schematic diagram showing an example of the mechanism of action of the SMAP method according to one embodiment of the present invention.
[FIG. 10] FIG. 10 is a schematic diagram showing an example of the mechanism of action of the SMAP method according to the embodiment of the present invention.

### Best Mode for Carrying Out the Invention

### <Isothermal Amplification DNA Polymerase>

The isothermal amplification DNA polymerase according to the present invention is, as described above, a DNA polymerase to be used in the isothermal amplification method of the present invention and composed of a protein described in any of the following items (a) to (d). The isothermal amplification method of the present invention to which the DNA polymerase of the present invention is applied will be described later.
(a) a protein having an amino acid sequence represented by SEQ ID NO. 23
(b) a protein having an amino acid sequence represented by SEQ ID NO. 25
(c) a protein having an amino acid sequence obtained by deletion of any number from 1 to 334 of consecutive amino acid residues starting from an N-terminal in the amino acid sequence represented by SEQ ID NO. 23,
(d) a protein having an amino acid sequence obtained by deletion, substitution, insertion, or addition of one or more amino acids in the amino acid sequence of the protein described in any of the items (a) to (c) and having a DNA polymerase activity

The isothermal amplification DNA polymerase according to the present invention can be isolated from, for example, microorganisms of the genus *Alicyclobacillus,* preferably from *Alicyclobacillus acidocaldarius (A. acidocaldarius*), and more preferably from A. *acidocaldarius* JCM 5260. This strain (JCM 5260) can be purchased from RIKEN (independent administrative institution) BioResource Center, Japan Collection of Microorganisms (http://www.jcm.riken.jp/JCM/Ordering_J.shtml). The isothermal amplification DNA polymerase according to the present invention hereinafter is also referred to as "DNA polymerase of the present invention", "DNA polymerase I of the present invention", "Aac polymerase of the present invention", or "protein of the present invention".

The DNA polymerase represented by SEQ ID NO. 23 in the item (a) can be isolated as a full-length DNA polymerase from A. *acidocaldarius* JCM 5260, for example. The DNA polymerase represented by SEQ ID NO. 25 in the item (b) is a protein having an amino acid sequence obtained by deletion of 1st to 334th amino acid residues starting from the N-terminal in the amino acid sequence represented by SEQ ID NO. 23 in the item (a). The protein having an amino acid sequence obtained by deletion of an N-terminal region formed of 1st to 334th amino acids in the amino acid sequence of SEQ ID NO. 23 as described above still has a DNA polymerase activity. Furthermore, the protein described in the item (c) is a protein having an amino acid sequence obtained by deletion of any number from 1 to 334 of consecutive amino acid residues starting from an N-terminal in the amino acid sequence represented by SEQ ID NO. 23. The protein having an amino acid sequence in which any number of amino acid residues are deleted in the above-described N-terminal region as described above still has a DNA polymerase activity. The number of the deleted amino acid residues is not particularly limited, and is, for example, in the range from 1 to 334. Furthermore, in the N-terminal region, consecutive amino acid residues may be deleted, or non-consecutive amino acid residues may be deleted.

As shown in the item (d), the DNA polymerase of the present invention may be a protein having an amino acid sequence obtained by deletion, substitution, insertion, or addition of one or more amino acids in the amino acid sequence described in any of the items (a) to (c), as long as it has a DNA polymerase activity. The expression "one or more amino acids" means, for example, about 5% to 10% of the number of the amino acid residues of the protein described in any of the items (a) to (c), and is, for example, about 1 to 50, preferably about 1 to 20, more preferably about 1 to 10, and most preferably about 1 to 5.

Furthermore, the DNA polymerase of the present invention may be a protein having a homology of, for example, at least 50% to the amino acid sequence of the protein described in any of the items (a) to (c) as shown in the following item (e), as long as it has a DNA polymerase activity. The homology preferably is at least 70%, at least 80%, at least 85%, at least 90%, at least 97%, and at least 98%. (e) a protein having an amino acid sequence with a homology of, for example, at least 50% to the amino acid sequence of the protein described in any of the items (a) to (c) and having a DNA polymerase activity.

Usually, the degree of the homology of two proteins can be indicated as a percentage of the identity between amino acid sequences of the proteins when they are aligned appropriately, and it represents the occurrence ratio of perfect match between the amino acid sequences. The appropriate alignment between the sequences for comparison of the identity can be determined using one of various algorithms, for example, a BLAST algorithm (Altschul, S. F. et al., J. Mol. Biol., 1990, Vol. 215, No.3, pp. 403-410).

The DNA polymerase of the present invention has, as polymerase activities, a template-dependent DNA replication activity and a complementary strand displacement replication activity, which are both ordinary activities, and it further has, for example, a reverse transcriptase activity. The DNA polymerase of the present invention further may have a 3' → 5' exonuclease activity. When the DNA polymerase of the present invention has a 3' → 5' exonuclease activity, the occurrence of error at the time of incorporating a substrate can be reduced further, for example. It is preferable that the DNA polymerase of the present invention exhibit an activity at, for example, any temperature from 25°C to 75°C, more preferably from 37°C to 72°C, still more preferably from 50°C to 70°C, and particularly preferably from 55°C to 65°C. The optimum temperature for the DNA polymerase of the present invention is, for example, higher than the optimum temperature (e.g., 20°C to 37°C) for known DNA polymerases having a complementary strand displacement replication activity. Specifically, for example, it is preferable that the DNA polymerase of the present invention exhibit the activity at any temperature from 25°C to 75°C, more preferably from 37°C to 72°C, still more preferably from 50°C to 70°C, and particularly preferably from 55°C to 65°C. Therefore, the DNA polymerase of the present invention can be used under reaction conditions with a temperature higher than that for the conventional DNA polymerases having a complementary strand displacement replication activity, for example. Thus, in specific isothermal amplification methods in the present invention including the SMAP method and LAMP method to be described later and the like, the DNA polymerase of the present invention can be used under more strict annealing conditions for a template DNA and a primer. Moreover, in the case where the DNA polymerase of the present invention has a reverse transcriptase activity, for example, it can be used for DNA synthesis using RNA as a template, and thus can be used in a method alternative to conventional RT-PCR.

Furthermore, the isothermal amplification DNA polymerase of the present invention includes, for example, an N-terminal deleted DNA polymerase in which an N-terminal side amino acid residue(s) is deleted as described above. The number of the N-terminal side amino acid residues to be deleted is not particularly limited, as long as, for example, the resultant N-terminal deleted DNA polymerase has a complementary strand displacement replication activity as described above. As a specific example, the N-terminal deleted DNA polymerase preferably is one that lacks a 5' → 3' exonuclease activity and has, for example, activities corresponding to those of Klenow fragment of DNA polymerase I from *Escherichia coli.* Such a DNA polymerase may be, for example, a protein having an amino acid sequence obtained by deletion of any number from 1 to 334 of consecutive amino acid residues starting from an N-terminal in the amino acid sequence represented by SEQ ID NO. 23, as shown in the item (b) or (c). Conceivably, when a relatively large number of consecutive amino acid residues starting from the N-terminal are deleted, the possibility that the DNA polymerase might lack the 5' → 3' exonuclease activity is relatively high. In DNA polymerases, a 5' → 3' exonuclease domain, a 3' → 5' exonuclease domain, and a polymerase domain are arranged in this order from the N-terminal side. Accordingly, when deleting the 5' → 3' exonuclease activity, this can be achieved by, for example, deleting any number of amino acid residues from the N-terminal side in the amino acid sequence of SEQ ID NO. 23 until the 5' → 3' exonuclease activity is no longer exhibited. In the amino acid sequence of SEQ ID NO. 23, a region formed of 62nd to 306th amino acids is considered to be a 5' → 3' exonuclease domain and a region formed of 392nd to 536th amino acids is considered to be a 3' → 5' exonuclease domain. However, the present invention is not limited thereto. As the isothermal amplification DNA polymerase according to the present invention, SEQ ID NO. 25 shows a specific example of the amino acid sequence of an N-terminal deleted DNA polymerase that has a DNA polymerase activity and a 3' → 5' exonuclease activity corresponding the activities of *Escherichia coli* Klenow fragment and lacks a 5' → 3' exonuclease activity. When the DNA polymerase of the present invention lacks the 5' → 3' exonuclease activity as described above, there is an advantage in that, during gene amplification, the amplification product obtained can be prevented from being degraded, for example.

Furthermore, the isothermal amplification DNA polymerase of the present invention may lack the 3' → 5' exonuclease activity. From the comparison of amino acid sequences of various kinds of DNA polymerase I, it has been known that, in the enzymes having the 3' → 5' exonuclease activity, three common sequence motifs are present in the central portion of the enzyme proteins. Enzymes from which these motifs are removed can be prepared. For example, commercially available Bst DNA polymerase derived from *Bacillus stearothermophilus* lacks the 3'→ 5' exonuclease activity (Aliotta, J. M. et al., Genetic Analysis: Biomolecular Engineering, Vol. 12, pp. 185-195, 1996). The DNA polymerase lacking the 3' → 5' exonuclease activity does not degrade the 3' end of a primer when it is used in an isothermal amplification reaction, for example. Thus, it brings about an advantage in that the reduction in primer concentration is prevented sufficiently, thus allowing a nucleic acid to be amplified more rapidly. Moreover, the DNA polymerase lacking the 3' → 5' exonuclease activity is suitable for use in, for example, the detection of point mutation. According to the DNA polymerase lacking the 3' → 5' exonuclease activity, it is possible to prevent sufficiently, for example, a primer from being degraded from its 3' side. Thus, the primer can discriminate sufficiently the mutation site of the template DNA, so that, for example, erroneous proceeding of the extension reaction can be suppressed and the detection of point mutation based on the termination of the extension reaction can be performed more accurately. As described above, in DNA polymerases, the 5' → 3' exonuclease domain, the 3' → 5' exonuclease domain, and a polymerase domain are arranged in this order from the N-terminal side. Accordingly, when deleting the 3' → 5' exonuclease activity, this can be achieved by, for example, deleting any number of amino acid residues in the amino acid sequence of SEQ ID NO. 23 or SEQ ID NO. 25 until the 3' → 5' exonuclease activity no longer is exhibited with the DNA polymerase activity being maintained. Furthermore, in the amino acid sequence of SEQ ID NO. 23, a region formed of 392nd to 536th amino acids is considered to be a 3' → 5' exonuclease domain. Thus, for example, this region may be deleted.

On the other hand, it has been suggested that, for the expression of the 3' → 5' exonuclease activity, for example, two metal ions coordinated in a carboxyl group in a side chain of an amino acid as an active center are important. Thus, for example, even when a DNA polymerase has a low 3' → 5' exonuclease activity, the enzyme activity can be enhanced by introducing an amino acid having a carboxyl group in its side chain into the active center (Park, Y. et al., Mol Cells. Vol. 7, No.3, pp. 419-424, 1997).

A method for measuring the activities of the DNA polymerase is not limited, and they can be measured by various measurement methods well known to those skilled in the art. Among the DNA polymerase activities, the template-dependent DNA replication activity can be measured using a fluorometric measurement method described in the literature (Seville M. et al. Biotechniques Vol. 21, pp. 664-668 (1996)) or the like, for example. Furthermore, among the DNA polymerase activities, the complementary strand displacement replication activity can be measured by the measurement method described in, for example, the above-described Non-Patent Document 1 (Kornberg, A. and Baker TA. DNA Replication, W.H. Freeman and Company, New York, 1992.) or the like. Still further, among the DNA polymerase activities, the reverse transcriptase activity can be measured by using, for example, a commercially available kit (e.g., EnzChek (trademark) Reverse Transcriptase Assay Kit (E-22064), Molecular Probes (Invitrogen)) in accordance with the protocol thereof.

### <Isothermal Amplification Method>

The isothermal amplification method of the present invention is an isothermal amplification method for carrying out isothermal amplification of a target nucleic acid sequence in a nucleic acid sample. The method includes amplifying the target nucleic acid sequence isothermally in the presence of the DNA polymerase of the present invention composed of a protein described in any of the above-described items (a) to (d) using a first primer shown in the following (X).
(X) a primer that contains, in a 3' end portion, a sequence (Ac') that hybridizes to a sequence (A) of a 3' end portion of the target nucleic acid sequence and also contains, on a 5' side of the sequence (Ac'), a sequence (B') that hybridizes to a complementary sequence (Bc) to a sequence (B) present on a 5' side with respect to the sequence (A) in the target nucleic acid sequence

The present invention provides the isothermal amplification method using the first primer shown in the above-described item (X). The method is characterized in that the isothermal amplification DNA polymerase of the present invention is used therein. Therefore, as long as the first primer shown in the item (X) and the isothermal amplification DNA polymerase according to the present invention are used in the method, other configurations, steps, conditions, etc. are not limited. Furthermore, according to the isothermal amplification reaction using the first primer shown in the item (X), a ladder-like amplification product is obtained at the time of electrophoretic analysis, for example. Thus, the isothermal amplification reaction to which the DNA polymerase of the present invention is applied also can be referred to as, for example, an isothermal amplification reaction for generating a ladder-like amplification product.

The isothermal amplification method generally is a method of carrying out a nucleic acid amplification reaction isothermally. The conditions for the amplification reaction are not particularly limited and can be determined as appropriate by those skilled in the art. Preferably, the reaction temperature is set at, for example, a temperature around the melting temperature (Tm) of the primer or lower. More preferably, the stringency level is set in view of the melting temperature (Tm) of the primer. Specific examples of the reaction temperature include about 20°C to about 75°C, preferably about 37°C to about 72°C, more preferably about 50°C to 70°C, and still more preferably about 55°C to 65°C.

When carrying out the amplification reaction, it is preferable to cause a mismatch binding protein to be present as well because it can improve specificity, for example. The mismatch binding protein (also referred to as a "mismatch recognition protein") is not limited, as long as it is a protein capable of recognizing a mismatch in, for example, a double-stranded nucleic acid and binding to a mismatch site thereof. For example, proteins known to those skilled in the art can be used. Furthermore, the mismatch binding protein may be, for example, a protein (a mutant) having an amino acid sequence obtained by substitution, deletion, addition, and/or insertion of one or more amino acids in an amino acid sequence of a wild-type protein, as long as it can recognize the mismatch in, for example, a double-stranded nucleic acid. Many mismatch binding proteins are known including, for example, a MutS protein (e.g., JP 9(1997)-504699 A), a MutM protein (e.g., JP 2000-300265 A), a MutS protein bonded to a green fluorescence protein (GFP) (WO 99/06591), Taq MutS, and analogs thereof (Radman, M. and Wagner, R., Annu. Rev. Genet. 20: 523-538 (1986); Radman, M. and Wagner, R., Sci. Amer., 1988, pp40-46; Modrich, P., J. Biol. Chem. 264: 6597-6600 (1989); Lahue, R. S. et al., Science 245: 160-164 (1988); Jiricny, J. et al,. Nucl. Acids Res. 16: 7843-7853 (1988); Su, S. S. et al., J. Biol. Chem. 263; 6829-6835 (1988); Lahue, R. S. et al., Mutat. Res. 198: 37-43 (1988); Dohet, C. et al., Mol. Gen. Gent. 206: 181-184 (1987); Jones, M. et al., Genetics 115:605-610 (1987); MutS of *Salmonella typhimurium* (Lu, A. L., Genetics 118: 593-600 (1988); Haber L. T. et al., J. Bacteriol. 170: 197-202 (1988); Pang, P. P. et al., J. Bacteriol. 163: 1007-1015 (1985)); Priebe S. D. et al., J. Bacterilo. 170: 190-196 (1988); and the like). In the present invention, preferable mismatch binding proteins include MutS, MSH2, MSH6, MutH, MutL, and one derived from yeast, for example.

Preferably, the aforementioned mismatch binding protein is being activated by an activator, for example, in order to prevent it from binding to a double-stranded nucleic acid containing no mismatch. The activator is not particularly limited. Examples thereof include ATP (adenosine 5'-triphosphate), ADP (adenosine 5'-diphosphate), ATP-γ-S (adenosine 5'-O-(3-thiotriphosphate)), and AMP-PNP (adenosine 5'-[β, γ-imide]triphosphate). Furthermore, the activator may be one of the nucleotides that can bind to a mismatch binding protein. A mismatch binding protein can be activated by incubating the mismatch binding protein and the activator at room temperature for several seconds to several minutes.

In the case where the mismatch binding protein is used, it is preferable to further use a single-stranded binding protein (SSB), for example, in order to prevent the mismatch binding protein from binding to a single-stranded nucleic acid. The SSB is not particularly limited and conventionally known proteins can be used. Specific examples of the SSB include single-stranded binding proteins derived from *Escherichia coli, Drosophila,* and *Xenopus laevis,* gene 32 proteins derived from T4 Bacteriophage, and in addition, those proteins derived from other species. In this case, examples of the mismatch binding protein include MutS, MutH, MutL, HexA, MSH1 to MSH6, Rep3, RNaseA, uracil-DNA glycosidase, T4 endonuclease VII, and resolvase. The mismatch binding protein is preferably MutS, MSH2, MSH6, or a mixture of two or more of them, and is more preferably MutS.

Examples of the isothermal amplification method using the first primer shown in the (X) include: LAMP (Loop-Mediated Isothermal Amplification) methods disclosed in WO 00/28082, Notomi, T et. al., Nucleic Acids Research (2000), Vol. 28, e63., and the like; and methods disclosed in Japanese Patent No. 3867926, Japanese Patent No. 3897805, Japanese Patent No. 3942627, and NATURE METHODS (Vol. 4, No. 3, March 2007, pp. 257-262), Mitani Y., Lezhava A., Kawai Y., Kikuchi T., Oguchi-Katayama A., Kogo Y., Itoh M., Miyagi T. et al. 2007. "Rapid SNP diagnostics using asymmetric isothermal amplification and a new mismatch-suppression technology." Nat. Methods 4 (3): 257-262. (hereinafter referred to as "SMart Amplification Process methods").

In the isothermal amplification method of the present invention, a second primer may be used in combination with the first primer shown in the (X). It is preferable that the first primer and the second primer are a pair of primers. Examples of the pair of primers include an asymmetric pair in which the first primer and the second primer are different from each other in morphology and a symmetric pair in which the first primer and the second primer are identical to each other in morphology. Hereinafter, a primer set including the asymmetric pair of primers also is referred to as an asymmetric primer set, and a primer set including the symmetric pair of primers also is referred to as a symmetric primer set. The asymmetric primer set is suitable for, for example, the SMart Amplification Process method, and the symmetric primer set is suitable for, for example, the LAMP method.

The isothermal amplification method of the present invention will be described with reference to an example where the method is carried out using the above-described asymmetric primer set and an example where the method is carried out using the above-described symmetric primer set. It is to be noted, however, the present invention is not limited thereto.

### SMart Amplification Process Method

Among isothermal amplification methods, the SMart Amplification Process method can amplify a target nucleic acid sequence with excellent specificity, for example. Accordingly, the SMart Amplification Process method makes it possible to judge, for example, the presence or absence of a mutation in a gene, particularly the presence or absence of single nucleotide mutation or the presence or absence of base deletion or base insertion by gene amplification.

As described above, the asymmetric primer set is a primer set having an asymmetric pair of primers composed of the first primer and the second primer that are different from each other in morphology. Particularly, it is preferable that the asymmetric primer set is used for the SMart Amplification Process method. Hereinafter, this primer set also is referred to as a "SMart Amplification Process primer set".

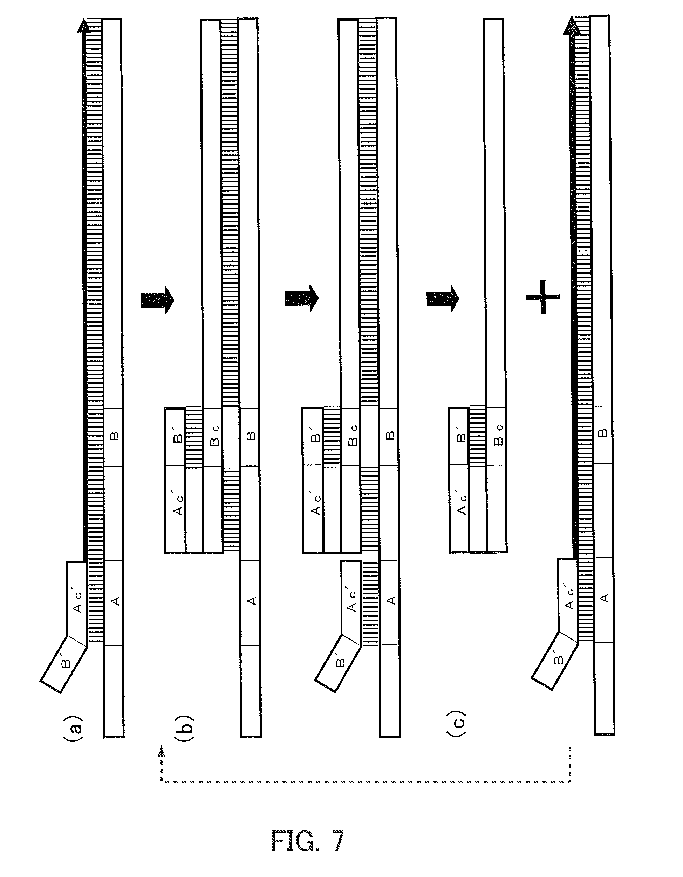

FIG. 7 schematically shows the action mechanism of nucleic acid synthesis to be conducted using the first primer. First, a target nucleic acid sequence contained in a nucleic acid to serve as a template is determined. Then, the sequence (A) that is located in the 3' end portion of the target nucleic acid sequence as well as the sequence (B) that is present on the 5' side with respect to the sequence (A) is determined. The first primer contains the sequence (Ac') and further contains the sequence (B') on the 5' side of the sequence (Ac'). The sequence (Ac') hybridizes to the sequence (A) while the sequence (B') hybridizes to the complementary sequence (Bc) to the sequence (B). In this case, the first primer may contain an intervening sequence that does not affect the reaction, between the sequence (Ac') and the sequence (B').
Annealing of such a primer to the template nucleic acid results in a state where the sequence (Ac') of the primer has hybridized to the sequence (A) of the target nucleic acid sequence (FIG. 7(a)). When a primer extension reaction occurs in this state, a nucleic acid containing the complementary sequence to the target nucleic acid sequence is synthesized. Then the sequence (B') that is present on the 5' end side of the nucleic acid thus synthesized hybridizes to the sequence (Bc) that is present in the nucleic acid. This allows a stem-loop structure to be formed in the 5' end portion of the synthesized nucleic acid. As a result, the sequence (A) located on the template nucleic acid becomes a single strand and then another primer having the same sequence as that of the preceding first primer hybridizes thereto (FIG. 7(b)). Thereafter, an extension reaction occurs from the newly hybridized first primer due to the strand displacement reaction. At the same time, the nucleic acid synthesized previously is dissociated from the template nucleic acid (FIG. 7(c)).

In the action mechanism described above, the phenomenon in which the sequence (B') hybridizes to the sequence (Bc) typically occurs due to the presence of the complementary regions on the same strand. Generally, when a double-stranded nucleic acid is dissociated into a single strand, partial dissociation starts from the ends thereof or from the relatively unstable portions other than the ends. In the double-stranded nucleic acid produced through the extension reaction caused by the above-mentioned first primer, base pairs located in the end portion are in a state of equilibrium between dissociation and binding at relatively high temperatures and thereby a double strand is retained as a whole. In such a state, when a sequence complementary to the dissociated portion located at the end is present on the same strand, a stem-loop structure can be formed in a metastable state.
This stem-loop structure does not exist stably. However, another identical primer binds to the complementary strand portion (the sequence (A) on the template nucleic acid) exposed due to the formation of the stem-loop structure, and thereby a polymerase causes the extension reaction immediately. Accordingly, while the strand synthesized previously is displaced and thereby is released, a new double-stranded nucleic acid can be produced at the same time.

The design criteria for the first primer according to a preferred aspect of the present invention are as follows, for example. First, in order for a new primer to anneal to the template nucleic acid efficiently after a complementary strand to the template nucleic acid is synthesized through the extension of a preceding primer, it is necessary to allow the sequence (A) portion located on the template nucleic acid to be a single strand through the formation of the stem-loop structure at the 5' end of the complementary strand synthesized as described above. For that purpose, a ratio of (X-Y)/X is important. That is a ratio of the difference (X-Y) to the number X, wherein X denotes the number of bases contained in the sequence (Ac') and Y indicates the number of bases contained in the region flanked by the sequence (A) and the sequence (B) in the target nucleic acid sequence. However, the portion that is present on the 5' side with respect to the sequence (A) on the template nucleic acid and that is not associated with the hybridization of the primer is not required to be a single strand. Furthermore, in order for a new primer to anneal to the template nucleic acid efficiently, it is also necessary that the above-mentioned stem-loop structure is formed efficiently. For the efficient formation of the stem-loop structure, i.e. for efficient hybridization between the sequence (B') and the sequence (Bc), the distance (X+Y) between the sequence (B') and the sequence (Bc) is important. Generally, the optimal temperature for the primer extension reaction is a maximum of around 72°C. It is difficult to dissociate the extended strand over a long region at such low temperatures. Therefore, conceivably, in order for the sequence (B') to hybridize to the sequence (Bc) efficiently, it is preferable that a smaller number of bases exist between the two sequences. On the other hand, conceivably, in order for the sequence (B') to hybridize to the sequence (Bc) to allow the sequence (A) portion located on the template nucleic acid to be a single strand, it is preferable that a larger number of bases exist in the sequence (B').

From such factors as described above, the aforementioned first primer according to a preferred embodiment of the present invention is designed so that (X-Y)/X is, for example, at least -1.00, preferably at least 0.00, more preferably at least 0.05, and still more preferably at least 0.10 but is, for example, 1.00 or lower, preferably 0.75 or lower, more preferably 0.50 or lower, and still more preferably 0.25 or lower, in the case where no intervening sequence is present between the sequence (Ac') and the sequence (B') that compose the primer. Moreover, (X+Y) is preferably at least 15, more preferably at least 20, and still more preferably at least 30 but is preferably 50 or less, more preferably 48 or less, and still more preferably 42 or less.

When an intervening sequence (the number of bases contained therein is Y') is present between the sequence (Ac') and the sequence (B') that compose the primer, the first primer according to the preferred embodiment of the present invention is designed so that, for example, {X-(Y-Y)}/X is at least -1.00, preferably at least 0.00, more preferably at least 0.05, and still more preferably at least 0.10 but is, for example, 1.00 or lower, preferably 0.75 or lower, more preferably 0.50 or lower, and still more preferably 0.25 or lower. Moreover, (X+Y+Y') is, for example, at least 15, preferably at least 20, and more preferably at least 30 but is, for example, 100 or less, preferably 75 or less, and more preferably 50 or less.

Preferably, the aforementioned first primer has a strand length that enables base pairing with the target nucleic acid while allowing the necessary specificity to be maintained under given conditions, for example. The strand length of this primer is preferably 15 to 100 nucleotides and more preferably 20 to 60 nucleotides. The lengths of the sequence (Ac') and the sequence (B') that compose the first primer each are preferably 5 to 50 nucleotides and more preferably 7 to 30 nucleotides. Furthermore, an intervening sequence that does not affect the reaction may be inserted between the sequence (Ac') and the sequence (B') if necessary.

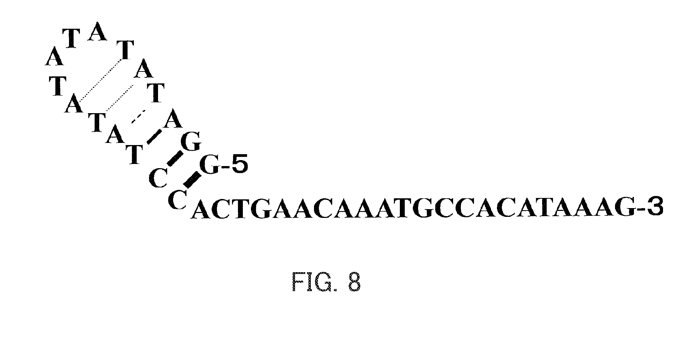

As described above, the second primer included in the primer set according to the present invention contains, for example, in its 3' end portion, a sequence (Cc') that hybridizes to a sequence (C) located in the 3' end portion of a complementary sequence (the strand located on the opposite side to the strand to which the first primer hybridizes) to the target nucleic acid sequence. The second primer also contains, on the 5' side of the sequence (Cc'), a folded sequence (D-Dc') that contains, on the same strand, two nucleic acid sequences that hybridize to each other. Such a second primer has a structure like the one shown in FIG. 8, for example. However, the sequence and the number of nucleotides of the second primer are not limited to those shown in FIG. 8. The length of the sequence (Cc') of the second primer is preferably 5 to 50 nucleotides and more preferably 10 to 30 nucleotides. On the other hand, the length of the folded sequence (D-Dc') is preferably 2 to 1000 nucleotides, more preferably 2 to 100 nucleotides, still more preferably 4 to 60 nucleotides, and even more preferably 6 to 40 nucleotides. The number of nucleotides of the base pairs that are formed through hybridization that occurs in the folded sequence is preferably 2 to 500 bp, more preferably 2 to 50 bp, still more preferably 2 to 30 bp, and even more preferably 3 to 20 bp. The nucleotide sequence of the folded sequence (D-Dc') may be any sequence and is not particularly limited. However, it is preferable that the nucleotide sequence is one that does not hybridize to the target nucleic acid sequence. In addition, an intervening sequence that does not affect the reaction may be inserted between the sequence (Cc') and the folded sequence (D-Dc') if necessary.

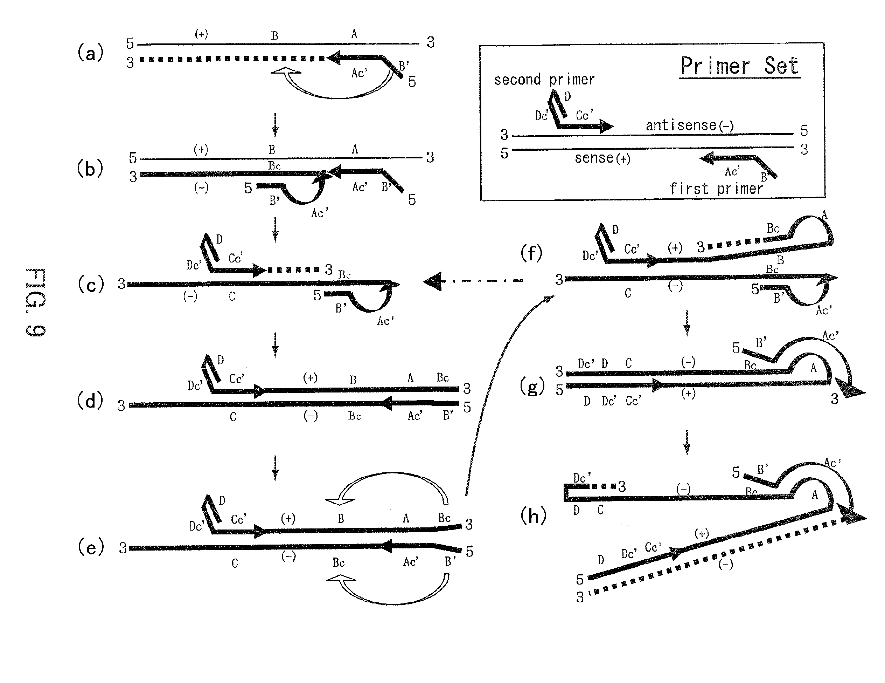

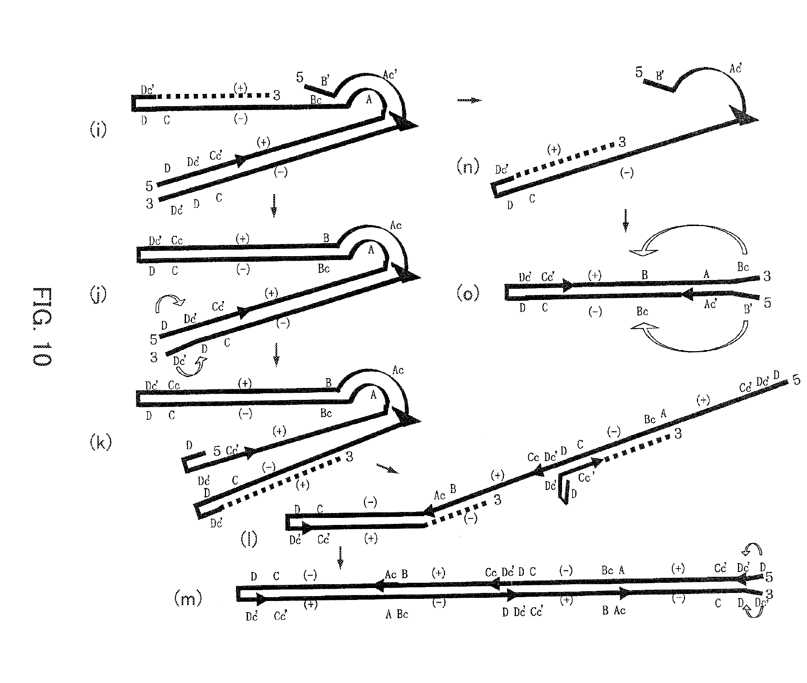

An example of the conceivable action mechanism of the isothermal amplification reaction that is caused by the above-mentioned first primer and second primer is described with reference to FIGs. 9 and 10. In FIGs. 9 and 10, in order to simplify the description, two sequences that hybridize to each other are described as sequences that are complementary to each other. It is to be noted, however, the present invention is not limited thereby. First, the first primer hybridizes to a sense strand of a target nucleic acid and thereby the extension reaction of that primer occurs (FIG. 9(a)). Subsequently, a stem-loop structure is formed on the extended strand (-) and thereby the sequence (A) on the target nucleic acid sense strand is allowed to be a single strand. Then a new first primer hybridizes to the sequence (A) (FIG. 9(b)). This causes the extension reaction of the primer, and then the extended strand (-) synthesized previously is dissociated. Next, the second primer hybridizes to the sequence (C) located on the dissociated extended strand (-) (FIG. 9(c)). This causes the extension reaction of the primer, and thereby an extended strand (+) is synthesized (FIG. 9(d)). Stem-loop structures are formed at the 3' end of the extended strand (+) thus synthesized and at the 5' end of the extended strand (-) (FIG. 9(e)). Then the extension reaction occurs from the loop end of the extended strand (+) that is the 3' end of the free form, and at the same time, the extended strand (-) is dissociated (FIG. 9(f)). The extension reaction that has occurred from the loop end results in production of a hairpin-type double-stranded nucleic acid to which the extended strand (-) has bound on the 3' side of the extended strand (+) through the sequence (A) and the sequence (Bc). Then the first primer hybridizes to the sequence (A) and the sequence (Bc) (FIG. 9(g)), and the extension reaction caused thereby allows the extended strand (-) to be produced (FIGs. 9(h) andFIG.10 (i)). Furthermore, the folded sequence that is present at the 3' end of the hairpin-type double-stranded nucleic acid provides the 3' end of the free form (FIG. 9(h)). Then, the extension reaction caused therefrom (FIG. 10(i)) allows a single-stranded nucleic acid to be produced (FIG. 10(j)). The single-stranded nucleic acid has the folded sequence at each end thereof and contains the extended strand (+) and the extended strand (-) alternately via the sequences derived from the first and second primers. In this single-stranded nucleic acid, the folded sequence that is present at the 3' end thereof provides the 3' end (the origin of complementary strand synthesis) of the free form (FIG. 10(k)). Accordingly, the similar extension reaction is repeated and the strand length is doubled per extension reaction (FIG. 10(l) and (m)). In the extended strand (-) synthesized from the first primer that has been dissociated in FIG. 10(i), the folded sequence that is present at the 3' end thereof provides the 3' end (the origin of complementary strand synthesis) of the free form (FIG. 10(n)). Accordingly, the extension reaction caused therefrom allows stem-loop structures to be formed at both ends and thereby a single-stranded nucleic acid is produced (FIG. 10(o)). The single-stranded nucleic acid contains the extended strand (+) and the extended strand (-) alternately via the sequences derived from the primers. Similarly in this single-stranded nucleic acid, the formation of a loop at the 3' end provides the origin of complementary strand synthesis consecutively. Accordingly, the extension reaction therefrom occurs in succession. In the single-stranded nucleic acid that is extended automatically in such a manner, the sequences derived from the first primer and the second primer are contained between the extended strand (+) and the extended strand (-). Therefore, each primer can hybridize to cause the extension reaction. This allows the sense strand and the antisense strand of the target nucleic acid to be amplified considerably.

The SMart Amplification Process primer set may include a third primer in addition to the first primer and the second primer. The third primer hybridizes to, for example, the target nucleic acid sequence or the complementary sequence thereto. However, the third primer does not compete with other primers for hybridization to the target nucleic acid sequence or the complementary sequence thereto. In the present invention, the expression "does not compete" means that hybridization of the third primer to a target nucleic acid does not hinder other primers from providing origins for complementary strand synthesis.

When the target nucleic acid sequence has been amplified with the first primer and the second primer, the amplification product contains the target nucleic acid sequence and the complementary sequence thereto alternately as described above. The amplification product has, at its 3' end, a folded sequence or a loop structure. It provides the origin of complementary strand synthesis and thereby extension reactions occur consecutively therefrom. It is preferable that when such an amplification product becomes a single strand partially, the third primer can anneal to the target sequence that is present in the single strand portion. This allows the target nucleic acid sequence contained in the amplification product to be provided with a new origin of complementary strand synthesis. Then an extension reaction occurs therefrom. Thus the isothermal amplification reaction is performed much quicker.

The third primer is not limited and may be of one kind, or for example, in order to improve the speed and specificity of the isothermal amplification reaction, at least two kinds of third primers may be used simultaneously. Typically, such third primers have, for example, different sequences from those of the first primer and the second primer. However, each of the third primers may hybridize to a region, a part of which is hybridized by the first or second primer, as long as they do not compete with the first or second primer. The strand length of the third primer is preferably 2 to 100 nucleotides, more preferably 5 to 50 nucleotides, and further preferably 7 to 30 nucleotides.

The third primer is intended mainly to provide an auxiliary function to advance the isothermal amplification reaction caused by the first primer and the second primer much quicker. Hence, it is preferable that the third primer have a lower Tm than that of each 3' end of the first primer and the second primer. Furthermore, it is preferable that the amount of the third primer to be added to the amplification reaction solution is smaller than that of each of the first primer and the second primer to be added thereto, for example.

The above-described third primer can be one that allows an origin of complementary strand synthesis to be provided for a loop portion, with a template having a structure capable of forming the loop, as described in, for example, WO 02/24902. The third primer, however, is not limited thereto. That is, it can be any primer that provides an origin of complementary strand synthesis for any site as long as the site is within the target nucleic acid sequence, for example.

In the SMart Amplification Process primer set, for example, either one of the first primer or the second primer or both of them may be a labeled nucleic acid(s) labeled with, for example, a fluorescent dye or the like, or the third primer may be the labeled nucleic acid. Either the first primer or the second primer or both of them and the third primer may be the labeled nucleic acids.

When the isothermal amplification method of the present invention is used, for example, for the mutation detection method to be described later, it is preferable that the SMart Amplification Process primer set is designed as follows. That is, preferably, the SMart Amplification Process primer set is designed so that a nucleic acid sequence having a mutation at a target site (hereinafter referred to as a "mutated nucleic acid sequence") or a nucleic acid sequence having no mutation at the target site (hereinafter referred to as a "wild-type nucleic acid sequence") is a target nucleic acid sequence, and the site where the target mutation occurs is contained in the sequence (A), the sequence (B), or the sequence (C), or is located between the sequence (A) and the sequence (B) or between the sequence (A) and the sequence (C).

When using a primer set designed so that a nucleic acid sequence containing a mutation at the target site (mutated sequence) is a target nucleic acid sequence, for example, the presence of an amplification product after the isothermal amplification reaction indicates the presence of the mutated sequence, while the absence of or reduction in the amplification product indicates the absence of the mutated sequence. On the other hand, when using a primer set designed so that a nucleic acid sequence containing no mutation at the target site (wild-type sequence) is a target nucleic acid sequence, for example, the presence of an amplification product after the isothermal amplification reaction indicates the absence of the mutated sequence, while the absence of or reduction in the amplification product indicates the presence of the mutated sequence. The expression "reduction in the amplification product" means a reduction in the amount of the amplification product obtained as compared to the amount of the amplification product that is obtained when the target nucleic acid sequence is present in the nucleic acid sample.

Preferably, the primer set is designed so that the target site is contained in the sequence (A). In the case of using such a primer set, for example, when the target nucleic acid sequence (e.g., a wild-type sequence) is contained in the nucleic acid sample, the first primer anneals to the sequence (A) in the isothermal amplification reaction and thereby an amplification product is obtained. On the other hand, when a nucleic acid sequence (e.g., a mutated sequence) that is different from the target nucleic acid sequence is contained in the nucleic acid sample, it is difficult for the first primer to anneal to the sequence (A) in the isothermal amplification reaction. Accordingly, in this case, no amplification product is obtained or a considerably reduced amount of amplification product is obtained. Preferably, the sequence (Ac') contained in the first primer is a sequence that is complementary to the sequence (A).

Preferably, the primer set is one designed so that, for example, the target site is contained in the sequence (C). In the case of using such a primer set, for example, when the target nucleic acid sequence (e.g., a wild-type sequence) is contained in the nucleic acid sample, the second primer anneals to the sequence (C) in the isothermal amplification reaction and thereby an amplification product is obtained. On the other hand, when a nucleic acid sequence (e.g., a mutated sequence) that is different from the target nucleic acid sequence is contained in the nucleic acid sample, it is difficult for the second primer to anneal to the sequence (C) in the isothermal amplification reaction. Therefore, in this case, no amplification product is obtained or a considerably reduced amount of amplification product is obtained. Preferably, the sequence (Cc') contained in the second primer is a sequence that is complementary to the sequence (C).

Preferably, the primer set is one designed so that, for example, the target site is contained in the sequence (B). In the case of using such a primer set, for example, when the target nucleic acid sequence (e.g., a wild-type sequence) is contained in the nucleic acid sample, after the first primer anneals to the sequence (A) to cause an extension reaction, a sequence (B') that is contained in the primer hybridizes to a sequence (Bc) located on the extended strand in the isothermal amplification reaction. Therefore a stem-loop structure is formed efficiently. This efficient formation of the stem-loop structure allows another first primer to anneal to the template. Accordingly, the action mechanism shown in FIG. 7 proceeds efficiently and thereby an amplification product is obtained. On the other hand, when a nucleic acid sequence (e.g., a mutated sequence) that is different from the target nucleic acid sequence is contained in the nucleic acid sample, it is difficult to form the above-mentioned stem-loop structure in the isothermal amplification reaction. Thus, the action mechanism shown in FIG. 7 is hindered. As a result, no amplification product is obtained or a considerably reduced amount of amplification product is obtained. Preferably, the sequence (B') contained in the first primer is a sequence identical to the sequence (B).

Preferably, the primer set is one designed so that, for example, the target site is located between the sequence (A) and the sequence (B). In the case of using such a primer set, when the target nucleic acid sequence (e.g., a wild-type sequence) is contained in the nucleic acid sample, after the first primer anneals to the sequence (A) to cause an extension reaction, the sequence (B') that is contained in the primer hybridizes to the sequence (Bc) located on the extended strand and thereby a stem-loop structure is formed efficiently in the isothermal amplification reaction. This efficient formation of the stem-loop structure allows another first primer to anneal to the template. Accordingly, the action mechanism shown in FIG. 7 proceeds efficiently and thereby an amplification product is obtained. On the other hand, when a nucleic acid sequence (e.g., a mutated sequence) that is different from the target nucleic acid sequence is contained in the nucleic acid sample, it is difficult to form the above-mentioned stem-loop structure in the isothermal amplification reaction since the distance maintained between the sequence (B') that is contained in the first primer and the sequence (Bc) located on the extended strand is not appropriate. This, for example, is the case where there is insertion or deletion of a long sequence between the sequence (A) and the sequence (B). Thus, in this case, the action mechanism shown in FIG. 7 is hindered. As a result, no amplification product is obtained or a considerably reduced amount of amplification product is obtained.

Preferably, the primer set is one designed so that the target site is located between the sequence (A) and the sequence (C). In the case of using such a primer set, when the target nucleic acid sequence is contained in the nucleic acid sample (e.g., a wild-type sequence), after the first primer anneals to the sequence (A) to cause an extension reaction, the sequence (B') that is contained in the primer hybridizes to the sequence (Bc) located on the extended strand and thereby a stem-loop structure is formed efficiently in the isothermal amplification reaction. This efficient formation of the stem-loop structure allows another first primer to anneal to the template. Accordingly, the action mechanism shown in FIGs. 7, 9, and 10 proceeds efficiently and thereby an amplification product is obtained. On the other hand, when a nucleic acid sequence (e.g., a mutated sequence) that is different from the target nucleic acid sequence is contained in the nucleic acid sample, no amplification product is obtained or a considerably reduced amount of amplification product is obtained. For instance, when the nucleic acid sample contains a nucleic acid sequence that is different from the target nucleic acid sequence due to the insertion of a long sequence between the sequence (A) and the sequence (C), the rate (efficiency) of isothermal amplification decreases considerably. As a result, no amplification product is obtained or a considerably reduced amount of amplification product is obtained. Furthermore, when the nucleic acid sample contains a nucleic acid sequence that is different from the target nucleic acid sequence due to the deletion of a sequence between the sequence (A) and the sequence (C) and when a part or the whole of the sequence (B) has been lost due to the deletion, the sequence (B') contained in the first primer cannot hybridize onto the extended strand. Accordingly, a stem-loop structure cannot be formed or forms with difficulty. Thus, the action mechanism shown in FIGs. 7, 9, and 10 is hindered. As a result, no amplification product is obtained or a considerably reduced amount of amplification product is obtained. Moreover, when the nucleic acid sample contains a nucleic acid sequence that is different from the target nucleic acid sequence due to the deletion of a sequence between the sequence (A) and the sequence (C) and when no partial deletion of the sequence (B) is caused by the deletion, the rate (efficiency) of isothermal amplification decreases. As a result, no amplification product is obtained or a considerably reduced amount of amplification product is obtained.

### LAMP method

As described above, the symmetric primer set includes a symmetric pair of primers composed of the first primer and the second primer that are identical to each other in morphology. Particularly, it is preferable that the primer set is used for the LAMP method. Hereinafter, this primer set also is referred to as a "LAMP primer set".

In the case where the first primer is designed, for example, so as to anneal to an antisense strand, the second primer is designed so as to anneal to a sense strand. In the case where the first primer is designed, for example, so as to anneal to a sense strand, the second primer is designed so as to anneal to an antisense strand. When the first primer has annealed to, for example, a target nucleic acid sequence in an antisense strand, it is preferable that the second primer anneal to a region in a sense strand corresponding to a 5' side region with respect to the site of the antisense strand to which the first primer has annealed.

In the LAMP method, for example, four kinds of primers are necessary. They recognize six regions, so that a target gene can be amplified. That is, in this method, for example, a first primer anneals to a template strand to cause an extension reaction first. Subsequently, the extended strand produced by the first primer separates from the template strand due to the strand displacement reaction caused by a second primer designed upstream from the first primer. At this time, a stem-loop structure is formed in the 5' end portion of the extended strand due to the structure of the first-primer extension product that has been removed. Similar reactions occur in the other strand of the double-stranded nucleic acid or on the 3' end side of the first-primer extension product that has been removed. These reactions are repeated and thereby the target nucleic acid is amplified. The template used in the LAMP method has, for example, at the 3' end and the 5' end on the same strand, regions composed of base sequences complementary to each other in the respective end regions. With this template (also referred to as a "dumbbell-type template nucleic acid"), loops are formed, in which base pairing can occur between the base sequences complementary to each other when they anneal to each other. The LAMP method can be performed according to, for example, WO 00/28082 or WO 01/034838.

### <Isothermal Amplification Kit>

The isothermal amplification kit of the present invention is a kit to be used in the isothermal amplification method of the present invention. The kit is characterized in that it includes the isothermal amplification DNA polymerase according to the present invention. According to the kit of the present invention, for example, the above-described isothermal amplification method of the present invention can be carried out easily and conveniently.
It is only necessary that the isothermal amplification kit of the present invention include the DNA polymerase of the present invention, and configurations and the like other than this are not limited. The above-described other configurations and the like can be determined as appropriate depending on the above-described isothermal amplification method. The isothermal amplification kit of the present invention may include, for example, a primer or a primer set, and a user manual in addition to the DNA polymerase of the present invention. The primer and the primer set may be those described above. Furthermore, the isothermal amplification kit of the present invention may further include: for example, a substrate such as dNTP mix (dATP, dTTP, dCTP, and dGTP); a buffer such as a Tris-HCl buffer, a tricine buffer, a sodium phosphate buffer, or a potassium phosphate buffer; a catalyst such as magnesium chloride, magnesium acetate, or magnesium sulfate; an additive such as dimethyl sulfoxide (DMSO) or betaine (N,N,N-trimethylglycine); an acidic substance and a cation complex described in WO 99/54455; an enzyme stabilizer; etc. The enzyme stabilizer is not limited. Examples thereof include glycerol, bovine serum albumin, and saccharide. Particularly, the enzyme stabilizer is preferably saccharide, more preferably monosaccharide or oligosaccharide, and still more preferably trehalose, sorbitol, mannitol, or a mixture of two or more of them. The isothermal amplification kit of the present invention further may include a melting temperature regulator. Examples of the melting temperature regulator include DMSO, betaine, formamide, glycerol, and arbitrary combinations thereof. It preferably is DMSO. Furthermore, when the nucleic acid sample used for the isothermal amplification method contains RNA and the RNA is used as a template, it is preferable that the kit further contain a reverse transcriptase, for example. In the isothermal amplification kit of the present invention, the ratio of these reagents, etc. are not limited and can be determined as appropriate by those skilled in the art.

The isothermal amplification kit of the present invention preferably further includes the above-described mismatch binding protein, and it preferably includes the single-stranded binding protein (SSB) in addition to the mismatch binding protein.

In the isothermal amplification kit of the present invention, reagents such as the DNA polymerase and the primer may be contained separately in different containers or may be contained in the same container. The isothermal amplification kit of the present invention also can be referred to as, for example, an isothermal amplification reagent to be used in the isothermal amplification method of the present invention.

### <DNA Encoding Isothermal Amplification DNA Polymerase>

In microorganisms of the genus *Bacillus* as the related genus, generally, a phoR gene is conserved upstream and a mutM gene is conserved downstream of a gene encoding DNA polymerase I (polA gene). Thus, a DNA polymerase gene (polA) of, for example, the genus *Alicyclobacillus* can be isolated by, for example, determining the base sequences of the conserved genes, designing a polA cloning primer based on the base sequences thus determined, and carrying out gene amplification. A specific example will be given below. It is to be noted, however, the present invention is not limited thereto.

First, from the conserved regions of the phoR gene and the mutM gene of a microorganism of the genus *Bacillus,* a primer or one or more pairs of primers for amplifying these genes are designed. Using such a primer set, each of the genes is amplified with a genomic DNA of a target bacterium (e.g., a microorganism of the genus *Alicyclobacillus*) being a template. Then, the base sequences of the resultant amplification products are determined.

Owing to the genomic structures of various microorganisms of the genus *Bacillus,* the phoR gene, the polA gene, and the mutM gene are transcribed in the same direction. Thus, based on the information as to the thus-determined base sequences, a primer (forward primer) homologous to a sense strand (complementary to an antisense strand) of the phoR gene and a primer (reverse primer) homologous to an antisense strand (complementary to a sense strand) of the mutM gene are designed as specific primers for polA cloning. Then, using these primers, DNA containing the polA gene is amplified with the genomic DNA of the bacterium being a template. By cloning the resultant amplification product, it is possible to obtain DNA containing the polA gene derived from the target bacterium. The length of the primer is not limited, and can be adjusted as appropriate depending on various experimental conditions. It is, for example, 15 to 50 mer, preferably 18 to 40 mer, and most preferably 25 to 35 mer.

A method for isolating a DNA polymerase gene from a bacterium belonging to or related to the genus *Alicyclobacillus* using such primers may include, for example, the following steps (A) to (D). The method may further include the following step (E).
(A) amplifying a DNA fragment of a phoR gene using a primer having a base sequence represented by SEQ ID NO. 1 or 2 and a primer having a base sequence represented by any of SEQ ID NOs. 3 to 5 with a genomic DNA extracted from the bacterium being a template
(B) amplifying a DNA fragment of a mutM gene using a primer having a base sequence represented by any of SEQ ID NOs. 6 to 8 and a primer having a base sequence represented by any of SEQ ID NOs. 9 to 11 with the genomic DNA extracted from the bacterium being a template
(C) determining a base sequence of each of the DNA fragments amplified in the steps (A) and (B)
(D) amplifying a DNA fragment containing a polA gene using a primer having a partial sequence of a sense strand of the phoR gene and a primer having a partial sequence of an antisense strand of the mutM gene, which are designed based on the base sequences determined in the step (C), with the genomic DNA extracted from the bacterium being a template
(E) cloning the DNA fragment amplified in the step (D)

The length of the primer is not limited, and can be adjusted as appropriate depending on various experimental conditions. It is, for example, 15 to 50 mer, preferably18 to 40 mer, and most preferably 25 to 35 mer. In the step (A), the primer having the base sequence represented by SEQ ID NO. 1 or 2 is a forward primer, and the primer having the base sequence represented by any of SEQ ID NOs. 3 to 5 is a reverse primer. In the step (B), the primer having the base sequence represented by any of SEQ ID NOs. 6 to 8 is a forward primer, and the primer having the base sequence represented by any of SEQ ID NOs. 9 to 11 is a reverse primer.

As the primer set in the step (D), for example, it is possible to use a forward primer having a base sequence represented by any of SEQ ID NOs. 12 to 17 and a reverse primer having a base sequence represented by SEQ ID NO. 18 in combination as will be described later. Furthermore, as a primer set for amplifying a full-length translation region, it is possible to use, for example, the combination of a forward primer having a base sequence represented by SEQ ID NO. 19 and a reverse primer having a base sequence represented by SEQ ID NO. 21 as will be described later. Still further, as a primer set for amplifying a coding sequence of a DNA polymerase in which an N-terminal region is deleted, it is possible to use, for example, the combination of a forward primer having a base sequence represented by SEQ ID NO. 20 and a reverse primer having a base sequence represented by SEQ ID NO. 21 as will be described later.

Examples of the DNA encoding the isothermal amplification DNA polymerase according to the present invention include DNAs shown in the following items (a') to (d'), which encode the DNA polymerases shown in the above-described items (a) to (d), respectively.
(a') DNA having a base sequence represented by SEQ ID NO. 22
(b') DNA having a base sequence represented by SEQ ID NO. 24
(c') DNA having a base sequence obtained by deletion of any number from 1 to 334 of consecutive codons starting from the 5' end in the base sequence represented by SEQ ID NO. 22
(d') DNA that has a base sequence obtained by deletion, substitution, insertion, or addition of one or more bases in the base sequence described in any of the items (a') to (c') and encodes a protein having a DNA polymerase activity

The base sequence represented by SEQ ID NO. 22 is, for example, a DNA sequence encoding the amino acid sequence represented by SEQ ID NO. 23. The base sequence represented by SEQ ID NO. 24 is, for example, a DNA sequence encoding the N-terminal deleted DNA polymerase represented by SEQ ID NO. 25. Note here that the present invention is not limited to these sequences.

Furthermore, DNA used in the present invention may be, for example, a DNA shown in the following (e'), which encodes a DNA polymerase shown in the above-described item (e), as long as the DNA polymerase encoded thereby has a DNA polymerase activity. (e') DNA that has a base sequence with a homology of, for example, at least 80% to the base sequence represented by any of the items (a') to (c') and encodes a protein having a DNA polymerase activity.

The homology can be calculated using the BLAST or the like under default conditions, for example. The homology is, for example, at least 80% or more, preferably 90% or more, and still more preferably 95% or more. The present invention also includes DNA that has a base sequence with a homology of, for example, at least 80% or more, preferably 90% or more, and more preferably 95% or more to DNA having the base sequence represented by SEQ ID NO. 24 when the homology is calculated using the BLAST or the like under the default conditions and encodes a protein having the DNA polymerase activity but lacking the 5' → 3' exonuclease activity. Furthermore, the present invention also includes RNA to the above-described DNA, e.g., mRNA transcribed from the above-described DNA, or an antisense RNA.

Furthermore, DNA in the present invention may be DNA shown in the following item (f'), for example, as long as a polymerase encoded thereby has a DNA polymerase activity.
(f') DNA that hybridizes to DNA having the base sequence described in any of the items (a') to (c') under stringent conditions and encodes a protein having a DNA polymerase activity

In the item (f'), "hybridization under stringent conditions" means hybridization under experimental conditions well known to those skilled in the art, for example. Specifically, the term "stringent conditions" refers to conditions such that a hybrid formed is identified after carrying out hybridization at 60°C to 68°C in the presence of 0.7 to 1 mol/l NaCl and then carrying out washing at 65°C to 68°C using a 0.1- to 2-fold SSC solution. Note here that 1 x SSC is composed of 150 mmol/l NaCl and 15 mmol/l sodium citrate, for example. In order to select the stringency, for example, the salt concentration and the temperature in the washing step can be optimized as appropriate. Furthermore, it is common general technical knowledge in the art to add, for example, formamide, SDS, or the like to improve the stringency.

Furthermore, DNA in the present invention includes, for example, a degenerate variant thereof having the base sequence represented by SEQ ID NO. 22 or 24. Still further, the present invention includes DNA having a complementary sequence to the above-described DNA.

Although a method for introducing mutation in a gene is not limited, the introduction of mutation can be carried out by, for example, a known method such as the Kunkel method or the gapped duplex method, or a method equivalent thereto. Also, it is possible to use a mutation introducing kit utilizing site-directed mutagenesis (e.g., Mutant-K (TAKARA), Mutant-G (TAKARA), or the like), LA PCR in vitro Mutagenesis series kit (TAKARA), or the like, for example.

### <Primer and Probe for DNA Isolation>

Next, a primer and a probe used for isolating a DNA polymerase from a living organism will be described. The primer and the probe each are, for example, a fragment of any of the above-described DNA of the present invention, and the number of bases is, for example, 5 to 50. The lower limit of the number of bases is, for example, 5 or more, preferably 10 or more, and more preferably 15 or more, and the upper limit of the number of bases is, for example, 50 or less, preferably 30 or less, and more preferably 25 or less. Furthermore, a specific examples of a preferable range of the number of bases is, for example, from 10 to 50, preferably from 10 to 30, and more preferably from 15 to 25. The length of a base sequence to be amplified is not particularly limited. Specific examples of the primer of the present invention include those represented by SEQ ID NOs. 1 to 21. One of them may be used alone, or two or more of them may be used in combination. In particular, it is preferable that at least one forward primer selected from the primers represented by SEQ ID NOs. 12 to 17 and a reverse primer that is the primer represented by SEQ ID NO. 18 are used in combination. When isolating a full-length translation region (ORF) of the polA gene, it is preferable that, for example, a forward primer represented by SEQ ID NO. 19 and a reverse primer represented by SEQ ID NO. 21 are used in combination. Furthermore, when isolating a region corresponding to *Escherichia coli* Klenow fragment in the polA gene, it is preferable that, for example, a forward primer represented by SEQ ID NO. 20 and a reverse primer represented by SEQ ID NO. 21 are used in combination.

### (Recombinant Vector)

Next, a recombinant vector that can be used for expression of the isothermal amplification DNA polymerase according to the present invention will be described. The recombinant vector is characterized in that it contains any of the above-described DNAs of the present invention. The recombinant vector can be obtained by, for example, ligating (inserting) the DNA of the present invention to a suitable vector. The vector to which the DNA is inserted is not particularly limited, as long as, for example, it can be replicated in a host, and examples thereof include plasmid DNA and phage DNA. Examples of the plasmid DNA include plasmids derived from *Escherichia coli* (e.g., pBR322, pBR325, pUC118, pUC119, and the like), plasmids derived from *Bacillus subtilis* (e.g., pUB110, pTP5, and the like), and plasmids derived from yeasts (e.g., YEp13, YEp24, YCp50, and the like). Examples of the phage DNA include λ phage (e.g., Charon 4A, Charon 21A, EMBL3, EMBL4, λgt10, λgt11, λZAP, and the like). Also, it is possible to use an animal virus such as retrovirus or vaccinia virus, an insect virus vector such as baculovirus, or the like.

A method for inserting the DNA into a vector is not particularly limited, and a conventionally known method can be employed. Specific examples include a method in which, for example, a purified DNA first is cleaved with a suitable restriction enzyme and the resultant DNA fragment is inserted into a restriction enzyme site or a multicloning site of a suitable vector DNA, thus ligating the DNA fragment to the vector. It is preferable that the DNA of the present invention is incorporated in the vector so as to allow the expression of a protein encoded by the DNA, for example. Thus, to the vector used in the present invention, for example, not only a promoter (e.g., a trp promoter, a lac promoter, a PL promoter, a tac promoter, or the like) but also a cis element such as an enhancer, a splicing signal, a poly-A addition signal, a selection marker, a ribosome binding sequence (an SD sequence, a KOZAK sequence, or the like), and the like may be ligated, if desired. Examples of the selection marker include a dihydrofolate reductase gene, an ampicillin resistance gene, and a neomycin resistance gene.

### (Transformant)

A transformant that can be used for the production of an isothermal amplification DNA polymerase according to the present invention will be described. The transformant is characterized in that it contains the above-described recombinant vector. The transformant is obtained by, for example, transfecting the above described DNA into a host so that the isothermal amplification DNA polymerase according to the present invention can be expressed. Specifically, it is preferable to transfect the recombinant vector of the present invention. In many cases, a vector is used for transformation because it allows the transformation to be carried out easily and efficiently, for example. The host is not particularly limited as long as it can express the protein of the present invention. Examples of the host include bacteria or the like belonging to: the genus *Escherichia* such as *Escherichia coli;* the genus *Bacillus* such as *Bacillus subtilis;* the genus *Pseudomonas* such as *Pseudomonas putida;* and the genus *Rhizobium* such as *Rhizobium meliloti.* In addition, yeasts such as *Saccharomyces cerevisiae* and *Schizosaccharomyces pombe,* animal cells such as a COS cell and CHO cell also can be used. Alternatively, it is also possible to use an insect cell such as Sf-9 and Sf-21, for example.

A method for carrying out the transformation is not limited, and a conventionally known method can be employed. Specific examples include a method using calcium ions (Cohen, S. N. et al. (1972) Proc. Natl. Acad. Sci., USA 69, 2110-2114), a DEAE dextran method, and an electroporation method.

### <Method for Producing Isothermal Amplification DNA Polymerase>

The isothermal amplification DNA polymerase according to the present invention can be produced by, for example, culturing the transformant and collecting a protein (DNA polymerase) from the resultant culture. The term "culture" means, for example, not only culture supernatant but also cultured cells or cultured bacterial cells and a homogenate thereof. Furthermore, "a method for culturing the transformant of the present invention" is carried out in accordance with, for example, a usual method applied to the culture of the host, and the conditions etc. thereof can be determined as appropriate depending on the kind of the host, etc., for example.

After the culture, in the case where the protein (DNA polymerase) of the present invention is produced in the bacterial cells or the cells, the protein is extracted by, for example, homogenizing the bacterial cells or the cells. On the other hand, in the case where the protein (DNA polymerase) of the present invention is produced outside the bacterial cells or the cells, for example, the culture solution is used as it is, or the bacterial cells or the cells are removed from the culture solution by centrifugation or the like. Thereafter, the protein (DNA polymerase) of the present invention can be purified from the culture by using a biochemical method commonly used for isolation and purification of proteins either alone or, if necessary, in combination with another method. Examples of the method for conducting the isolation and purification include ammonium sulfate precipitation, gel chromatography, ion exchange chromatography, and affinity chromatography. Furthermore, for example, in the case where a protein to be expressed has a tag sequence for purification, the tag sequence can be removed by a protease treatment or the like during or after the purifying step.

### <Antibody>

Furthermore, an antibody against the DNA polymerase of the present invention will be described. The term "antibody" encompasses, for example, any suitable fragments and derivatives. Examples of the antibody in such a broad sense include polyclonal antibodies, monoclonal antibodies, a Fab fragment, a Fab' fragment, a F(ab')₂ fragment, a Fv fragment, diabody (two copies of the same Fv fragment, fused to each other), single-chain antibodies, and multi-specific antibodies each composed of two or more antibody fragments. The antibody can be used for, for example, purification of the isothermal amplification DNA polymerase according to the present invention. The method for producing the antibody is not limited, and the antibody can be produced by a conventionally known method, for example.

In the present invention, for example, general methods based on molecular biology, microbiology, a recombinant DNA technology, and the like can be conducted with reference to standard reference books in the art. Examples of these books include: Molecular Cloning: A Laboratory Manual, Third Edition (Sambrook & Russell, Cold Spring Harbor Laboratory Press, 2001); Current Protocols in Molecular Biology (edited by Ausubel et al., John Wiley & Sons, 1987); a series of Methods in Enzymology (Academic Press); PCR Protocols: Methods in Molecular Biology (edited by Bartlett & Stirling, Humana Press, 2003); and Antibodies: A Laboratory Manual (edited by Harlow & Lane, Cold Spring Harbor Laboratory Press, 1987). Furthermore, reagents, kits, etc. referred to herein are available from commercial vendors such as, for example, Sigma, Aldrich, Invitrogen/GIBCO, Clontech, Stratagene, Qiagen, Promega, Roche Diagnostics, Becton-Dickinson, and Takara Bio Inc.

The present invention will be described in further detail with reference to the following examples. It is to be noted, however, the present invention is by no means limited to these examples.

### Example 1

### [Cloning of Alicyclobacillus acidocaldarius (A. acidocaldarius) DNA polymerase I gene (polA)]

From cultured bacterial cells of A. *acidocaldarius* subsp. *Acidocaldarius* JCM 5260, genomic DNA was prepared according to a conventional method. Based on the fact that phoR is conserved upstream of polA and mutM is conserved downstream of the polA in polA gene structures of other species of the genus *Bacillus,* primers shown below were synthesized from the conserved regions of the respective genes.

### phoR cloning PCR primers

### phoRFwd1 (x6144):

5'-AARMARYTWGARSARRTKMGVAA-3' (SEQ ID NO. 1) phoRFwd2 (x576):
5'-GTHTCYCATGARYTRAARACDCC-3' (SEQ ID NO. 2) phoRRev1 (x1296):
5'-HCKRTARAAVCGTTCRAADATVC-3' (SEQ ID NO. 3) phoRRev2 (x1728):
5'-GTHCCDCCHGWRTTKCKRCTYCT-3' (SEQ ID NO. 4) phoRRev3 (x6912):
5'-RYHARRTGCTTBACRATYGMHAR-3' (SEQ ID NO. 5)

### mutM cloning PCR primer

### mutMFwd1 (x96):

5'-TGCCDGAATTACCRGARGTNGAR-3' (SEQ ID NO. 6) mutMFwd2 (x3072):
5'-GMMGRGGMAARTTYYTDYTKTTW-3' (SEQ ID NO. 7) mutMFwd3 (x1728):
5'-GTBWSHCAYYTKMGDATGGAAGG-3' (SEQ ID NO. 8) mutMRev1 (x288):
5'-AAAGAGGTGCATCGTTCCRAAYT-3' (SEQ ID NO. 9) mutMRev2 (x324):
5'-TCBACATADATRTTBCCVAGYCC-3' (SEQ ID NO. 10) mutMRev3 (x864):
5'-CCBCKYCCBCCAACRACHRTYTT-3' (SEQ ID NO. 11)

In the above sequences, R denotes a purine base of guanine or adenine, Y denotes a pyrimidine base of thymine or cytosine, M denotes adenine or cytosine, K denotes guanine or thymine, S denotes guanine or cytosine, W denotes adenine or thymine, D denotes adenine, guanine, or thymine, H denotes adenine, cytosine, or thymine, V denotes adenine, guanine, or cytosine, N denotes any one of four kinds of bases (guanine, adenine, cytosine, thymine). The number in parentheses denotes the number of the kinds of each primer obtained through combination. Furthermore, Fwd denotes a forward primer, and Rev denotes a reverse primer. The same applies to sequences described below.

Using the thus-prepared genomic DNA as a template, DNA amplification was carried out by PCR with the use of each combination of a forward (Fwd) primer and a reverse (Rev) primer. The amplified DNA fragment was cloned into Plasmid pGEM-T (trade name, Promega KK). Specifically, the DNA fragment of the phoR gene was amplified using either one of phoRFwd1 and phoRFwd2 as a forward primer and any one of phoRRev1, phoRRev2, and phoRRev3 as reverse primer. Furthermore, the DNA fragment of the mutM gene also was amplified using any one of mutMRev1, mutMRev2, and mutMRev3 as a forward primer and any one of mutMRev1, mutMRev2, and mutMRev3 as a reverse primer. Then, each of the thus-obtained DNA fragments was cloned into the plasmid, and the base sequence inside thereof was determined.

Based on the base sequences thus determined, the following primers for cloning a polA gene were synthesized.
Aac cloning F1:
   TTTATCCACCTTGAGCGGCACAGACCAGTT (SEQ ID NO. 12)
Aac cloning F2:
   TTCGCACCTTCCACTGGCTCTCTGCACCGC (SEQ ID NO. 13)
Aac cloning F3:
   GACGTACTCTCTCCTTCATGGCCTTCGCTC (SEQ ID NO. 14)
Aac cloning F4:
   AATTTTGTGAACATCATAATCAATTCGTTG (SEQ ID NO. 15)
Aac cloning F5:
   CCACAAGACGACGCGGGCCGACAAGGGGAA (SEQ ID NO. 16)
Aac cloning F6:
   TGGCCTTCGCTCGATGAATTTTGTGAACAT (SEQ ID NO. 17)
Aac cloning R1:
   GGTGAATGCCCTGCTCCCTCAGCCGCTCGG (SEQ ID NO. 18)

Using the prepared genomic DNA as a template, DNA amplification was carried out by PCR with the use of the combination of any one of the Aac cloning F1 to F6 as a forward primer and the Aac cloning R1 as a reverse primer. The amplified DNA fragment was cloned into Plasmid pGEM-T (trade name, Promega KK). Then, the base sequence of the amplified DNA fragment cloned into the plasmid was determined. Subsequently, based on the thus-determined base sequence and the base sequence presumed to be the gene (polA) of *Alicyclobacillus acidocaldarius* DNA polymerase (Aac DNA polymerase I), the following primer pairs were designed. Using these primer pairs, a full-length translation region (ORF) moiety was amplified by PCR.
Aac polA NdeI:
   5'-CTTCATGGCCTTCGCcatATGAATTTTGTG-3' (SEQ ID NO. 19)
Aac term KpnI:
   5'-TCCGGCACGCCGgtaCCCCCCTCACTTGGC-3' (SEQ ID NO. 21)

The resultant PCR amplification product and a plasmid pYSN were digested with restriction enzymes NdeI and KpnI and were mixed together, whereby the PCR amplification product was ligated into the plasmid pYSN. Thus, an Aac DNA polymerase expression plasmid pAac was constructed.

Furthermore, from the thus-obtained Aac DNA polymerase expression plasmid pAac, the base sequence of the polA gene was determined. Then, a DNA fragment corresponding to the N-terminal of *Escherichia coli* Klenow fragment was amplified by PCR using the following primer pair.
Aac Klenow NdeI:
   5'-CGCGCCATCGCCTGGcatatgGAGCTCGAC-3' (SEQ ID NO. 20)
Aac term KpnI:
   5'-TCCGGCACGCCGgtaCCCCCCTCACTTGGC-3' (SEQ ID NO. 21)

The resultant PCR amplification product and a plasmid pYSN were digested with restriction enzymes NdeI and KpnI and mixed together, whereby the PCR amplification product was ligated into the plasmid pYSN. Thus, expression plasmid pdNAac for N-terminal deleted Aac DNA polymerase (hereinafter also referred to as "ΔN Aac DNA polymerase", "Aac DNA polymerase large fragment" or "Aac DNA polymerase I") was constructed.

### Example 2

### [Expression and purification of Aac DNA polymerase I and N-terminal deleted Aac DNA polymerase]

### (1) Culture, expression, and crude liquid extract preparation

*Escherichia coli* XL1-Blue having pAac or pdNAac was cultured in 5 ml of LB medium containing 100 µg/ml ampicillin at 37°C overnight. The resultant solution was used as a preculture solution. 5 ml of this preculture solution was inoculated into 500 ml of LB medium containing 100 µg/ml ampicillin, and was subjected to shaking culture at 37°C and 200 rpm (Orbital Shaking Incubator, FIRSTEK OSI-502LD). At the time when the OD 600 nm of the culture solution reached around 0.5, IPTG was added so that the final concentration thereof was 1 mmol/l. The resultant mixture was subjected to further shaking culture at 37°C and 200 rpm for 1 to 2 hours. Subsequently, the culture solution was transferred into a centrifuge tube and subjected to centrifugation at 4,000 × g for 10 minutes. As a result, a precipitate was obtained. This precipitate was suspended in 30 ml of 1 × PBS, and the resultant suspension again was centrifuged at 4,000 × g for 10 minutes, thereby washing bacterial cells. The resultant precipitate was suspended in 25 ml of 1 × PBS, and sonication of the cells for 10 seconds was carried out six times in total (MISONIX Astrason Ultrasonic processor XL). The sample after being subjected to the sonication was centrifuged at 15,000 × g for 30 minutes. Thus, a supernatant was obtained. To this supernatant, a 30% polyethyleneimine solution was added so that the final concentration thereof was 0.1%, and they were mixed together. The resultant mixture was allowed to stand in ice for 30 minutes and then was centrifuged at 15,000 × g for 30 minutes. Thus, a supernatant was obtained. The thus-obtained supernatant was used as a crude liquid extract.

### (2) Anion-exchange column chromatography

Ion exchange chromatography was performed using AKTA Prime high performance liquid chromatography system manufactured by GE Healthcare and an anion-exchange column HiTrapQ manufactured by GE Healthcare. As a running buffer, 50 mmol/l Tris-HCl (pH 7.6) containing 10 mmol/l 2-mercaptoethanol was used. The column was equilibrated at a flow rate of 1 ml/min, and the above-described crude liquid extract was applied thereto. Thereafter, a non-adsorbed fraction was washed with the running buffer. An adsorbed fraction was eluted with a concentration gradient (from 0 to 1 mol/l) of about 15 column volumes of sodium chloride. The eluted fraction was fractionated every 1 ml, and each fraction was applied to SDS-PAGE to check a protein band. Then, the fraction having a protein band of the corresponding molecular weight was collected. The collected fraction was concentrated and desalted using an ultrafilter membrane, and this was used as an anion-exchange fraction.

### (3) Heparin affinity column chromatography

Heparin affinity column chromatography was performed using AKTA Prime high performance liquid chromatography system manufactured by GE Healthcare and a heparin affinity column HiTrap Heparin manufactured by GE Healthcare. As a running buffer, the same solution as in the above-described anion-exchange column chromatography was used. The column was equilibrated at a flow rate of 1 ml/min, and the above-described anion-exchange fraction was applied thereto. Thereafter, a non-adsorbed fraction was washed with the running buffer. An adsorbed fraction was eluted with a concentration gradient (from 0 to 1 mol/l) of about 22 column volumes of sodium chloride. The eluted fraction was fractionated every 1 ml, and each fraction was applied to SDS-PAGE to check a protein band. Then, the fraction having a protein band of the corresponding molecular weight was collected. Using an ultrafilter membrane, the collected fraction then was subjected to buffer exchange with 50 mmol/l Tris-HCl (pH 8.0) containing 0.2 mol/l sodium chloride and further to concentration. This was used as a heparin fraction.

### (4) Gel filtration column chromatography

Gel filtration column chromatography was performed using AKTA 10XT high performance liquid chromatography system manufactured by GE Healthcare and a gel filtration column HiLoad 16/60 Superdex 200 prep grade manufactured by GE Healthcare. As a running buffer, 50 mmol/l Tris-HCl (pH 8.0) containing 0.2 mol/l sodium chloride was used. The column was equilibrated at a flow rate of 1 ml/min, and the above-described heparin fraction was applied thereto. Thereafter, elution was performed using the running buffer. The eluted fraction was fractionated every 1 ml, and each fraction was applied to SDS-PAGE to check a protein band. Then, the fraction having a protein band of the corresponding molecular weight was collected. The collected fraction was concentrated using an ultrafilter membrane, and buffer exchange with a storage buffer was performed. This was used as a purified enzyme preparation. The composition of the storage buffer is: 50 mmol/l potassium chloride, 10 mmol/l Tris-HCl (pH 7.5), 1mmol/l DTT, 0.1 mmol/l EDTA, 0.1% Triton X-100, and 50% glycerol.

### Example 3

### [DNA polymerase activity measurement]

A DNA polymerase activity of the purified enzyme preparation (Aac DNA polymerase large fragment) was measured using Picogreen dsDNA quantification reagent manufactured by Invitrogen with reference to Seville M. et al., BioTechniques Vol. 21, pp.664-668 (1996). Specifically, the Picogreen dsDNA quantification reagent and a TE buffer were mixed together at a volume ratio of 1 : 345. Then, 173 µl of the resultant mixture was added to 27 µl of a mixture of M13mp18 single-stranded DNA, a primer, dNTP, and the purified enzyme preparation. This reaction solution was left to stand at room temperature (37°C) for 5 minutes. Thereafter, the fluorescence thereof was measured at an excitation wavelength of 480 nm and a measurement wavelength of 520 nm. At this time, the fluorescence of a commercially available Klenow fragment (commercially available Bst DNA polymerase; available from NEB) with a known unit (unit: an amount of enzyme required for incorporating 10 nmol of dNTP at 65°C in 30 minutes) also was measured in the same manner. From the measured value thereof, an enzyme unit as a relative value was calculated. One example of the measurement is shown below. A standard curve was prepared for each measurement, and the reaction temperature was set to 37°C. Using the commercially available Bst DNA polymerase (available from NEB, hereinafter the same) as the standard, the fluorescence intensity was measured with respect to each dilution factor using the Picogreen dsDNA quantification reagent. The results thereof are shown in Table 1.

**[Table 1]**

| Bst DNA polymerase | Measurement 1 (rfu) | Measurement 2 (rfu) |
|---|---|---|
| 0.5 unit | 59.252 | 57.995 |
| 1.0 unit | 61.232 | 62.425 |
| 2.0 unit | 74.589 | 75.559 |
| 4.0 unit | 91.235 | 93.357 |
| 6.0 unit | 95.987 | 102.00 |
| 8.0 unit | 120.60 | 129.25 |

| | | |
|---|---|---|
| rfu: relative fluorescence units | | |

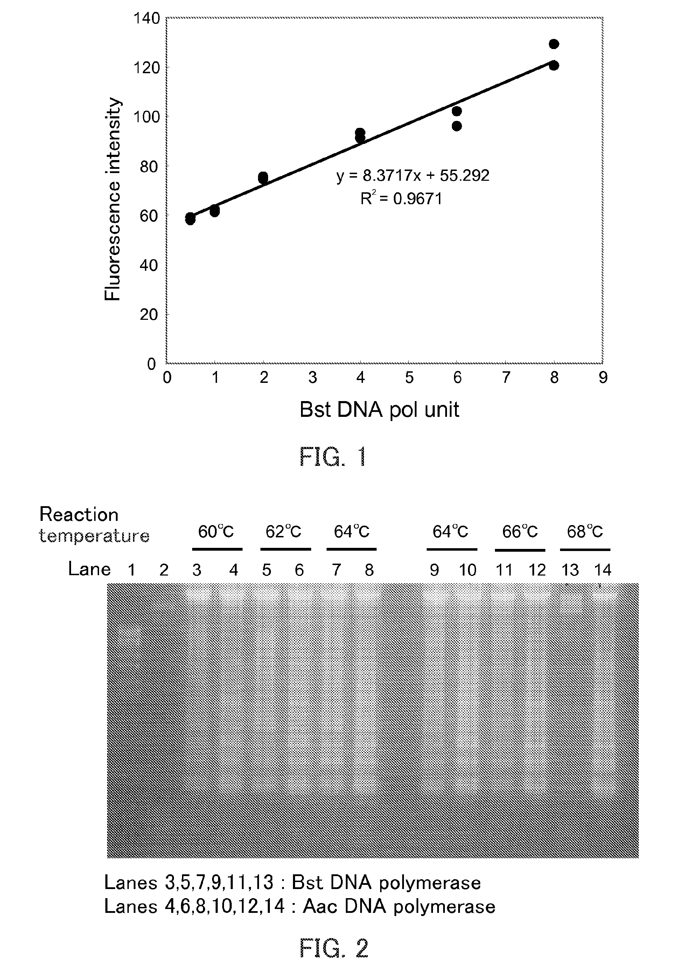

These results were plotted and regressed to a first-order linear equation. As a result, the following equation was obtained. The thus-obtained regression curve is shown in FIG. 1. In the following equation, x denotes a unit indicating a polymerase activity and y denotes a fluorescence intensity. Regarding the DNA polymerase activity, 1 unit corresponds to the amount of enzyme required for incorporating 10 nmol of dNTP into an acid-insoluble fraction at 65°C in 30 minutes. $y = 8.3717 ⁢ x + 55.292 \left(R⁢2=0.9671\right)$

Furthermore, the purified enzyme preparation (Aac DNA polymerase large fragment) subjected to the measurement exhibited an average fluorescence intensity of 98.03 (the first time: 97.789, the second time: 98.262) at the reaction temperature (room temperature: 37°C). Thus, from the above-described regression linear equation, the DNA polymerase activity thereof was calculated to be about 5.10 units at the reaction temperature. From this result, it was confirmed that the purified enzyme preparation obtained in Example 2 was a DNA polymerase.

### Example 4

### [Measurement of complementary strand displacement replication activity]

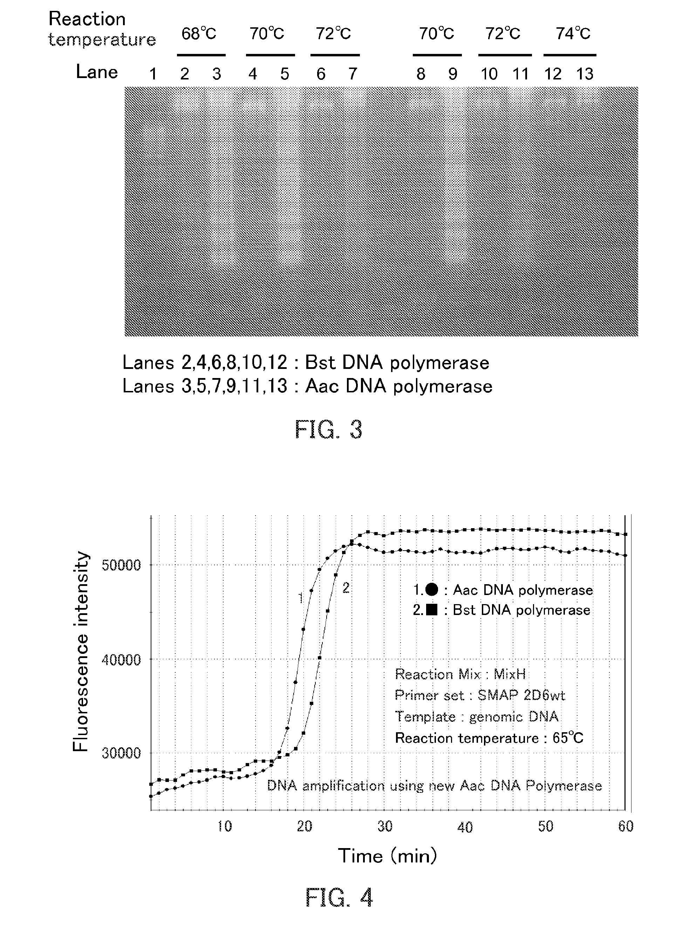

Activity measurement was carried out according to a method described in the above-described Non-Patent Document 2 (Notomi, T. et al., Nucleic Acids Research, 2000, Vol. 28, No. 12, e63). First, a mixture of each of synthetic DNAs (M13mp18 single-stranded DNA, 0.8 µmol/l FIP, 0.8 µmol/l BIP, 0.2 µmol/l F3, and 0.2µmol/l B3), 1 mol/l betaine, 20 mmol/l Tris-HCl buffer (pH 8.8), 10 mmol/l potassium chloride, 10 mmol/l ammonium sulfate, 0.1% Triton X-100, and 2-4 mmol/l magnesium sulfate was prepared in an amount of 20 µl. The mixture was left to stand at 95°C for 5 minutes and then on ice for 5 minutes. Then, 5 µl of the above-described purified enzyme preparation was added to the mixture, and the resultant mixture was left to stand at a predetermined reaction temperature (60°C to 74°C) for 1 hour. Thereafter, it was applied to agarose gel electrophoresis. At this time, Bst DNA polymerase (large fragment) with a known unit was applied to the electrophoresis in the same manner. Then, by comparing the band intensity of a purified product of the purified enzyme preparation after the electrophoresis and the band intensity of a purified product of the Bst DNA polymerase with a known unit after the electrophoresis, an enzyme unit as a relative value was calculated. Moreover, the electrophoresis also was carried out with respect to a blank obtained by adding the Tris-HCl buffer instead of the purified enzyme preparation (Aac DNA polymerase large fragment). The results thereof are shown in FIGs. 2 and 3.

FIGs. 2 and 3 are electrophoretograms of gene amplification products obtained through amplification at predetermined temperatures. In FIGs. 2 and 3, lane 1 shows a molecular weight marker (λ-Sty I). Lane 2 in FIG. 2 shows the result of the blank (containing no DNA polymerase). Odd-numbered lanes (3, 5, 7, 9, 11, 13) in FIG. 2 show the results obtained when the Bst DNA polymerase large fragment was used to perform the reaction at respective temperatures from 60°C to 68°C, whereas even-numbered lanes (4, 6, 8, 10, 12, 14) in FIG. 2 show the same when the purified enzyme preparation (Aac DNA polymerase large fragment) was used. Even-numbered lanes (2, 4, 6, 8, 10, 12) in FIG. 3 show the results obtained when the Bst DNA polymerase large fragment was added to perform the reaction at respective temperatures from 68°C to 74°C, whereas odd-number lanes (3, 5, 7, 9, 11, 13) in FIG. 3 show the same when the purified enzyme preparation (Aac DNA polymerase large fragment) was added. In FIGs. 2 and 3, the reaction temperature (°C) is shown for each lane.

As can be seen from FIGs. 2 and 3, it was confirmed that the purified enzyme preparation, i.e., the Aac DNA polymerase large fragment obtained in Example 2 had a complementary strand displacement replication activity. Furthermore, at the temperatures at and below 64°C, it exhibited the activity equivalent to that of the Bst DNA polymerase large fragment. Still further, at the temperatures at and above 66°C, in particular, at and above 68°C, the amplification amount decreased significantly in the case where the Bst DNA polymerase large fragment was used, whereas a sufficient activity was seen at the temperatures up to 72°C in the case where the Aac DNA polymerase large fragment was used. This result demonstrates that the Aac DNA polymerase large fragment is an enzyme that is stable at high temperature.

### Example 5

### [SMart Amplification Process method]

According to the SMart Amplification Process method (the method proposed by Mitani et al. For example, see Patent Document 4 (Japanese Patent No. 3867926) and Patent Document 5), isothermal amplification of a nucleic acid was performed. 10 µl of a reaction solution having the following composition was prepared, and this was reacted at 65°C for 60 minutes. This reaction was monitored in real time using Mx3000P (trade name, Stratagene). As a comparative example, the following reaction solution was prepared by adding a commercially available Bst DNA polymerase (large fragment) instead of the Aac DNA polymerase large fragment so that the final concentration thereof was 0.32 unit/µl, and the real time monitoring was conducted in the same manner. These results are shown in FIG. 4.

**[Table 2]**

| Reaction Composition | | |
|---|---|---|
| Tris-HCl (pH 8.8) | | 20 mmol/l |
| KCl | | 10 mmol/l |
| (NH₄)₂SO₄ | | 10 mmol/l |
| magnesium sulfate | | 6 mmol/l |
| Tween 20 | | 0.1% |
| betaine | | 0.8 mol/l |
| dNTPs | | 1.4 mmol/l |
| SYBR (registered trademark) Green I* | | 0.01 µl |
| genomic DNA** | | 1.6 ng/l |
| five kinds of primers | TP | 2.16 µmol/l |
| | FP | 2.16 µmol/l |
| | BP | 1.08 µmol/l |
| | OP1 | 0.135 µmol/l |
| | OP2 | 0.315 µmol/l |
| DNA polymerase | | 0.64 unit/µl |
| | | 25 µl |

| | | |
|---|---|---|
| *: Molecular Probes, Inc. **: Promega, Human Genomic DNA, Male | | |

### Primers

As a SMart Amplification Process primer set, the following five kinds of primers were provided. A first primer is Turn-back Primer (TP), a second primer is Folding Primer (FP), and third primers are Boost Primer for wild-type detection (BPw), Outer Primer 1 (OP1), and Outer Primer 2 (OP2).
TP 5'-CGCTGCACATGGCCTGGGGCCTCCTGCTCA-3' (SEQ ID NO. 26)
FP 5'-tttatatatatataaaCCCCTGCACTGTTTCCCAGA-3' (SEQ ID NO. 27)
BP 5'-ATCCGGATGTAGGATC-3' (SEQ ID NO. 28)
OP1 5'-GATGGTGACCACCTCGAC-3' (SEQ ID NO. 29)
OP2 5'-TGTACCCTTCCTCCCTCG-3' (SEQ ID NO. 30)

FIG. 4 is a graph showing the relationship between the number of cycles and fluorescence intensity obtained through real time monitoring of the isothermal amplification. In FIG. 4, a filled circle indicates the result obtained in the example using the Aac DNA polymerase large fragment, and a filled square indicates the result obtained in the comparative example using the commercially available Bst DNA polymerase large fragment. As can be seen from FIG. 4, in the example using the Aac DNA polymerase large fragment (filled circle), the target sequence was amplified more rapidly than in the comparative example using the commercially available Bst DNA polymerase large fragment (filled square). Moreover, by determining the base sequence of the amplification product, it was confirmed that the target sequence had been amplified. In the field of nucleic acid amplification, it is significant to shorten the time required for amplification even on a minute time scale. Therefore, it can be said that, by using the Aac DNA polymerase of the present invention, the time required for amplification can be shortened sufficiently relative to isothermal amplification methods using conventional DNA polymerases.

### Example 6

According to the SMart Amplification Process method using an Aac DNA polymerase large fragment, wild-type human ALDH2 was detected.

### (1) Nucleic acid sample

To 15 µl of blood containing hetero-type human ALDH2, 30µl of 50 mmol/l NaOH was added. The resultant mixture was heat-treated at 98°C for 3 minutes and then cooled with ice. This was used as a nucleic acid sample.

### (2) Primers

As a SMart Amplification Process primer set, the following five kinds of primers were provided. A first primer is Turn-back Primer (TP), a second primer is Folding Primer (FP), and third primers are Boost Primer for wild-type detection (BPw), Outer Primer 1 (OP1), and Outer Primer 2 (OP2).
- TP: 5'-CGAGTACGGGCCCACACTCACAGTTTTCAC-3' (SEQ ID NO. 31)
- FP: 5'-TTTATATATATATAAACCGGGAGTTGGGCGAG-3' (SEQ ID NO. 32)
- BPw: 5'-GCAGGCATACACTGA-3' (SEQ ID NO. 33)
- OP1: 5'-CCTGAGCCCCCAGCAGGT-3' (SEQ ID NO. 34)
- OP2: 5'-ACAAGATGTCGGGGAGTG-3' (SEQ ID NO. 35)

### (3) SMart Amplification Process reaction

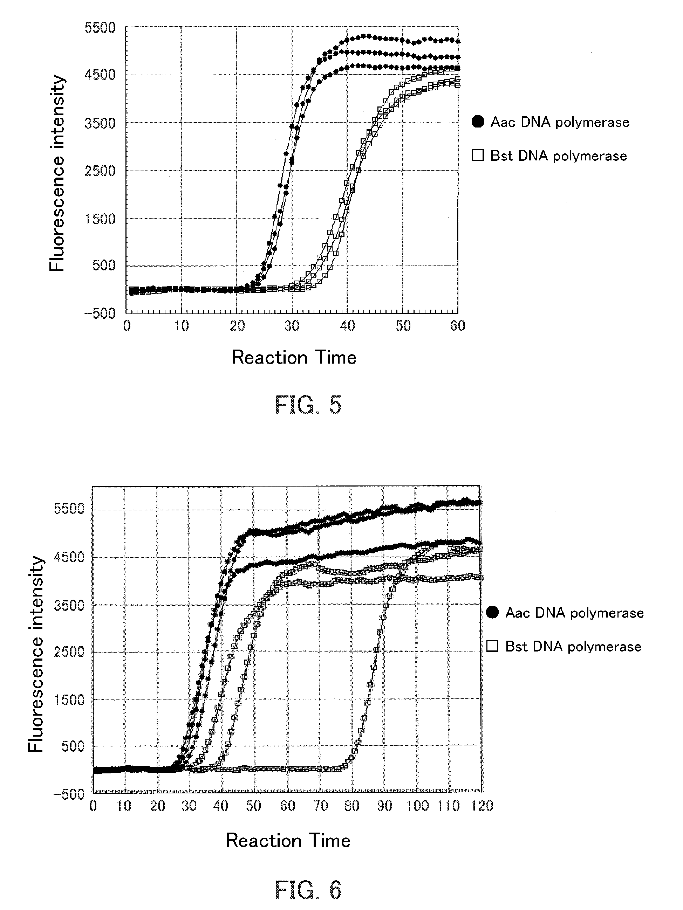

A reaction solution having the following composition (composition per 25 µl) was prepared in the total amount of 75 µl, and the reaction solution was reacted at 60°C for 1 hour. During the reaction, the fluorescence intensity of the reaction solution was monitored in real time with Mx3000P (trade name, Stratagene) while maintaining an isothermal condition at 60°C. As a comparative example, the reaction solution was prepared by adding 6 U of a commercially available Bst DNA polymerase (large fragment) instead of the Aac DNA polymerase large fragment, and the real time monitoring was conducted in the same manner. 75 µl of the reaction solution was divided equally into three, and the reaction measurement was conducted with respect to each of 25 µl of the equally-divided reaction solutions. These results are shown in FIG. 5. FIG. 5 is a graph showing an amplification profile obtained when an isothermal amplification reaction was carried out by the SMart Amplification Process method. A filled circle indicates the result obtained when the Aac DNA polymerase large fragment was used, and an open square indicates the result obtained when the commercially available Bst DNA polymerase large fragment was used.

**[Table 3]**

| Reaction Composition | | |
|---|---|---|
| Tris-HCl (pH 9.0, 25°C) | | 20 mmol/l |
| KCl | | 10 mmol/l |
| (NH₄)₂SO₄ | | 10 mmol/l |
| magnesium sulfate | | 8 mmol/l |
| Tween 20 | | 0.1% |
| dNTPs | | 1.4 mmol/l |
| DMSO | | 5% |
| SYBR (registered trademark) Green I* | | 1/100,000 dilution |
| nucleic acid sample** | | 1 µl |
| five kinds of primers | FP | 2.0 µmol/l |
| | TP | 2.0 µmol/l |
| | BP | 1.0 µmol/l |
| | OP1 | 0.25 µmol/l |
| | OP2 | 0.25 µmol/l |
| DNA polymerase | | 6 units |
| | | 25 µl |

| | | |
|---|---|---|
| *: Molecular Probes, Inc. **: The sample was added so that 6000 copies were present in each reaction system. | | |

As can be seen from FIG. 5, in the case where the Aac DNA polymerase large fragment was used, the target sequence was amplified more rapidly as compared with the case where the commercially available Bst DNA polymerase large fragment was used. Specifically, it was confirmed that the fluorescence intensity reached a plateau about 8 to 12 minutes earlier in the former case than in the latter case. Specifically, in the reaction system using the Aac DNA polymerase large fragment, amplification was observed within 30 minutes (filled circle). In contrast, in the reaction system using the Bst DNA polymerase large fragment, amplification was observed at and later than 30 minutes (open square). From this result, it can be said that the Aac DNA polymerase is an enzyme highly suitable for the SMart Amplification Process method as compared with the Bst DNA polymerase.

### Example 7

According to the LAMP method using an Aac DNA polymerase large fragment, wild-type human DIO2 was detected.

### (1) Nucleic acid sample

To 15 µl of blood containing hetero-type human DIO2, 30µl of 50 mmol/l NaOH was added. The resultant mixture was heat-treated at 98°C for 3 minutes and then cooled with ice. This was used as a nucleic acid sample.

### (2) Primers

As a LAMP primer sets, the following five kinds of primers were provided. TPw and TRFs2 form an asymmetric pair of primers.
- TPw: 5'-tactggagacGTGAAATTGGGTGAGGATGC-3' (SEQ ID NO. 36)
- TPFs2: 5'-AGAAGGAGGTgtaccattgccactgtt-3' (SEQ ID NO. 37)
- BP: 5'-cacactggaattggggg-3' (SEQ ID NO. 38)
- OP1: 5'-tcagctatcttctcctgg-3' (SEX ID NO. 39)
- OP2: 5'-TGTGATATTCTCACCTTC-3' (SEQ ID NO. 40)

### (3) LAMP reaction

A reaction solution having the following composition (composition per 25 µl) was prepared in the total amount of 75 µl, and the reaction solution was reacted at 60°C for 2 hours. During the reaction, the fluorescence intensity of the reaction solution was monitored in real time with Mx3000P (trade name, Stratagene) while maintaining an isothermal condition at 60°C. As a comparative example, the reaction solution was prepared by adding 6 U of a commercially available Bst DNA polymerase (large fragment) instead of the Aac DNA polymerase large fragment, and the real time monitoring was conducted in the same manner. 75 µl of the reaction solution was divided equally into three, and the reaction measurement was conducted with respect to each of 25 µl of the equally-divided reaction solutions. These results are shown in FIG. 6. FIG. 6 is a graph showing an amplification profile obtained when an isothermal amplification reaction was carried out by the LAMP method. A filled circle indicates the result obtained when the Aac DNA polymerase large fragment was used, and an open square indicates the result obtained when the commercially available Bst DNA polymerase large fragment was used.

**[Table 4]**

| Reaction Composition | | |
|---|---|---|
| MOPS (pH 7.4, 25°C) | | 20 mmol/l |
| KCl | | 25 mmol/l |
| (NH₄)₂SO₄ | | 10 mmol/l |
| magnesium sulfate | | 8 mmol/l |
| Tween 20 | | 0.1% |
| dNTPs | | 1.4 mmol/l |
| DMSO | | 5% |
| SYBR (registered trademark) Green I* | | 1/100,000 dilution |
| nucleic acid sample** | | 1 µl |
| five kinds of primers | FP | 2.0 µmol/l |
| | TP | 2.0 µmol/l |
| | BP | 1.0 µmol/l |
| | OP1 | 0.25 µmol/l |
| | OP2 | 0.25 µmol/l |
| DNA polymerase | | 6 units |
| | | 25 µl |

| | | |
|---|---|---|
| *: Molecular Probes, Inc. **: The sample was added so that 6000 copies were present in each reaction system. | | |

As can be seen from FIG. 6, in the case where the large fragment of the Aac DNA polymerase was used, it was confirmed that the amplification proceeds more rapidly as compared with the case where the large fragments of the commercially available Bst DNA polymerases were used. Specifically, in the reaction system in which the large fragment of the Aac DNA polymerase was used, amplification was observed within 30 minutes (filled circle). In contrast, in the reaction system in which the large fragment of Bst DNA polymerase was used, amplification was observed later as compared with the case where the Aac DNA polymerase (large fragment) was used, and there was a case where the amplification was observed later than 80 minutes (open square). This demonstrates that Aac DNA polymerase is more stable than Bst DNA polymerase and is highly suitable for the LAMP method.

### Industrial Applicability

As specifically described above, the isothermal amplification DNA polymerase of the present invention can improve the amplification efficiencies of the specific isothermal amplification methods such as the SMart Amplification Process method and the LAMP method as compared with conventional DNA polymerases used in the isothermal amplification methods. Thus, the isothermal amplification DNA polymerase of the present invention allows nucleic acid amplification to be carried out in a shorter time than ever before. Moreover, since the time required for nucleic acid amplification can be shortened, for example, analysis of single nucleotide polymorphism (SNP) or the like utilizing the nucleic acid amplification can be conducted more rapidly and a large amount of specimen can be analyzed more efficiently. Therefore, the present invention is very useful in the fields of nucleic acid analyses, clinical tests, and the like utilizing nucleic acid amplification.

## Claims

1. An isothermal amplification method for carrying out isothermal amplification of a target nucleic acid sequence in a nucleic acid sample, the method comprising:
carrying out an amplification reaction of the target nucleic acid sequence isothermally in the presence of a DNA polymerase comprising a protein described in any of the following items (a) to (d) using a first primer shown in the following (X):
(a) a protein having an amino acid sequence represented by SEQ ID NO. 23;
(b) a protein having an amino acid sequence represented by SEQ ID NO. 25;
(c) a protein having an amino acid sequence obtained by deletion of any number from 1 to 334 of consecutive amino acid residues starting from an N-terminal in the amino acid sequence represented by SEQ ID NO. 23;
(d) a protein having an amino acid sequence obtained by deletion, substitution, insertion, or addition of one or more amino acids in the amino acid sequence of the protein described in any of the items (a) to (c) and having a DNA polymerase activity; and
(X) a primer that contains, in a 3' end portion, a sequence (Ac') that hybridizes to a sequence (A) of a 3' end portion of the target nucleic acid sequence and also contains, on a 5' side of the sequence (Ac'), a sequence (B') that hybridizes to a complementary sequence (Bc) to a sequence (B) present on a 5' side with respect to the sequence (A) in the target nucleic acid sequence.

2. The isothermal amplification method according to claim 1, wherein the DNA polymerase has a DNA polymerase activity at least at any temperature in a range from 25°C to 75°C.

3. The isothermal amplification method according to claim 1, wherein the DNA polymerase has a complementary strand displacement replication activity as the DNA polymerase activity.

4. The isothermal amplification method according to claim 1, wherein the DNA polymerase has a reverse transcriptase activity as the DNA polymerase activity.

5. The isothermal amplification method according to claim 1, wherein the DNA polymerase lacks a 5' → 3' exonuclease activity.

6. The isothermal amplification method according to claim 1, wherein the DNA polymerase has a 3' → 5' exonuclease activity.

7. The isothermal amplification method according to claim 1, wherein the DNA polymerase lacks a 3' → 5' exonuclease activity.

8. The isothermal amplification method according to claim 1, wherein
a second primer is used in combination with the first primer in the amplification reaction, and
the first primer and the second primer are an asymmetric pair of primers different from each other in morphology.

9. The isothermal amplification method according to claim 8, wherein the second primer contains, in a 3' end portion, a sequence (Cc') that hybridizes to a sequence (C) of a 3'end portion of a complementary sequence to the target nucleic acid sequence and also contains, on a 5' side of the sequence (Cc'), a folded sequence (D-Dc') that contains, on the same strand, two nucleic acid sequences that hybridize with each other.

10. The isothermal amplification method according to claim 8, wherein
a third primer further is used in combination with the first primer and the second primer in the amplification reaction,
the third primer hybridizes to the target nucleic acid sequence or a complementary sequence thereto and does not compete with other primers for hybridization to the target nucleic acid sequence or the complementary sequence thereto, and
when an amplification product of the first primer or second primer is brought into a single-stranded state partially, the third primer can anneal to a target nucleic acid sequence present in a moiety that is in the single-stranded state, so that a new origin of complementary strand synthesis is provided for a target nucleic acid sequence in the amplification product.

11. The isothermal amplification method according to claim 1, wherein a second primer further is used in combination with the first primer in the amplification reaction, and
the first primer and the second primer are a symmetric pair of primers identical to each other in morphology.

12. The isothermal amplification method according to claim 11, wherein the primer set is for use in a LAMP method.

13. A DNA polymerase to be used in the isothermal amplification method according to claim 1, the DNA polymerase comprising a protein described in any of the following items (a) to (d):
(a) a protein having an amino acid sequence represented by SEQ ID NO. 23;
(b) a protein having an amino acid sequence represented by SEQ ID NO. 25;
(c) a protein having an amino acid sequence obtained by deletion of any number from 1 to 334 of consecutive amino acid residues starting from an N-terminal in the amino acid sequence represented by SEQ ID NO. 23; and
(d) a protein having an amino acid sequence obtained by deletion, substitution, insertion, or addition of one or more amino acids in the amino acid sequence of the protein described in any of the items (a) to (c) and having a DNA polymerase activity.

14. The isothermal amplification DNA polymerase according to claim 13, having a DNA polymerase activity at least at any temperature in a range from 25°C to 75°C.

15. The isothermal amplification DNA polymerase according to claim 13, having a complementary strand displacement replication activity as the DNA polymerase activity.

16. The isothermal amplification DNA polymerase according to claim 13, having a reverse transcriptase activity as the DNA polymerase activity.

17. The isothermal amplification DNA polymerase according to claim 13, lacking a 5' → 3' exonuclease activity.

18. The isothermal amplification DNA polymerase according to claim 13, having a 3' → 5' exonuclease activity.

19. The isothermal amplification DNA polymerase according to claim 13, lacking a 3' → 5' exonuclease activity.

20. An isothermal amplification kit to be used in the isothermal amplification method according to claim 1, the isothermal amplification kit comprising the isothermal amplification DNA polymerase according to claim 13.

21. The isothermal amplification kit according to claim 20, further comprising a first primer shown in the following (X):
(X) a primer that contains, in a 3' end portion, a sequence (Ac') that hybridizes to a sequence (A) of a 3' end portion of the target nucleic acid sequence and also contains, on a 5' side of the sequence (Ac'), a sequence (B') that hybridizes to a complementary sequence (Bc) to a sequence (B) present on a 5' side with respect to the sequence (A) in the target nucleic acid sequence.

22. The isothermal amplification kit according to claim 21, further comprising a second primer,
wherein the first primer and the second primer are an asymmetric pair of primers different from each other in morphology.

23. The isothermal amplification kit according to claim 21, further comprising a second primer,
the first primer and the second primer are a symmetric pair of primers identical to each other in morphology.


### [Sequence Listing]

SEQUENCE LISTING

<110>  RIKEN
       KABUSHIKI KAISHA DNAFORM

<120>  Method of isothermal amplification and DNA polymerase therefof

<130>  P29483-WO-EP

<140>  08 842 183.9
<151>  2008-10-24

<150>  JP2007/277496
<151>  2007-10-25

<160>  40

<170>  PatentIn version 3.1

<210>  1
<211>  23
<212>  DNA
<213>  Artificial

<220>
<223>  Forward primer 1 for cloning phoR

<400>  1
aarmarytwg arsarrtkmg vaa                                              23


<210>  2
<211>  23
<212>  DNA
<213>  Artificial

<220>
<223>  Forward primer 2 for cloning phoR

<400>  2
gthtcycatg arytraarac dcc                                              23


<210>  3
<211>  23
<212>  DNA
<213>  Artificial

<220>
<223>  Reverse primer 1 for cloning phoR

<400>  3
hckrtaraav cgttcraada tvc                                              23


<210>  4
<211>  23
<212>  DNA
<213>  Artificial

<220>
<223>  Reverse primer 2 for cloning phoR

<400>  4
gthccdcchg wrttkckrct yct                                              23


<210>  5
<211>  23
<212>  DNA

<213>  Artificial

<220>
<223>  Reverse primer 3 for cloning phoR

<400>  5
ryharrtgct tbacratygm har                                              23


<210>  6
<211>  23
<212>  DNA
<213>  Artificial

<220>
<223>  Forward primer 1 for cloning mutM

<220>
<221>  misc_feature
<222>  (20)..(20)
<223>  n is A, G, T, or C


<400>  6
tgccdgaatt accrgargtn gar                                              23


<210>  7
<211>  23
<212>  DNA
<213>  Artificial

<220>
<223>  Forward primer 2 for cloning mutM

<400>  7
gmmgrggmaa rttyytdytk ttw                                              23


<210>  8
<211>  23
<212>  DNA
<213>  Artificial

<220>
<223>  Forward primer 3 for cloning mutM

<400>  8
gtbwshcayy tkmgdatgga agg                                              23


<210>  9
<211>  23
<212>  DNA
<213>  Artificial

<220>
<223>  Reverse primer 1 for cloning mutM

<400>  9
aaagaggtgc atcgttccra ayt                                              23


<210>  10
<211>  23
<212>  DNA
<213>  Artificial

<220>
<223>  Reverse primer 2 for cloning mutM

<400>  10
tcbacatada trttbccvag ycc                                              23


<210>  11
<211>  23
<212>  DNA
<213>  Artificial

<220>
<223>  Reverse primer 3 for cloning mutM

<400>  11
ccbckyccbc caacrachrt ytt                                              23


<210>  12
<211>  30
<212>  DNA
<213>  Artificial

<220>
<223>  Forward primer 1 for cloning polA

<400>  12
tttatccacc ttgagcggca cagaccagtt                                       30


<210>  13
<211>  30
<212>  DNA
<213>  Artificial

<220>
<223>  Forward primer 2 for cloning polA

<400>  13
ttcgcacctt ccactggctc tctgcaccgc                                       30


<210>  14
<211>  30
<212>  DNA
<213>  Artificial

<220>
<223>  Forward primer 3 for cloning polA

<400>  14
gacgtactct ctccttcatg gccttcgctc                                       30


<210>  15
<211>  30
<212>  DNA
<213>  Artificial

<220>
<223>  Forward primer 4 for cloning polA

<400>  15
aattttgtga acatcataat caattcgttg                                       30


<210>  16
<211>  30
<212>  DNA
<213>  Artificial

<220>
<223>  Forward primer 5 for cloning polA

<400>  16
ccacaagacg acgcgggccg acaaggggaa                                        30


<210>  17
<211>  30
<212>  DNA
<213>  Artificial

<220>
<223>  Forward primer 6 for cloning polA

<400>  17
tggccttcgc tcgatgaatt ttgtgaacat                                        30


<210>  18
<211>  30
<212>  DNA
<213>  Artificial

<220>
<223>  Reverse primer 1 for cloning polA

<400>  18
ggtgaatgcc ctgctccctc agccgctcgg                                        30


<210>  19
<211>  30
<212>  DNA
<213>  Artificial

<220>
<223>  Forward primer for amplifying full-rength polA

<400>  19
cttcatggcc ttcgccatat gaattttgtg                                        30


<210>  20
<211>  30
<212>  DNA
<213>  Artificial

<220>
<223>  Forward primer for amplifying klenow-like fragment

<400>  20
cgcgccatcg cctggcatat ggagctcgac                                        30


<210>  21
<211>  30
<212>  DNA
<213>  Artificial

<220>
<223>  Reverse primer for amplifying polA

<400>  21
tccggcacgc cggtaccccc ctcacttggc                                        30


<210>  22
<211>  3137
<212>  DNA

<213>  Alicyclobacillus acidocaldarius

<220>
<221>  CDS
<222>  (69)..(2897)
<223>

<400>  22
ttcgcacctt ccactggctc tctgcaccgc cccggcggac gtactctctc cttcatggcc      60

ttcgctcg atg aat ttt gtg aac atc ata atc aat tcg ttg aag tgt gtc      110
         Met Asn Phe Val Asn Ile Ile Ile Asn Ser Leu Lys Cys Val
         1               5                   10

aac tcg gcg tgc gtc ccg ctg ggt gtg agg ccg ttt tcg tgc cgc cac      158
Asn Ser Ala Cys Val Pro Leu Gly Val Arg Pro Phe Ser Cys Arg His
15                  20                  25                  30

aag acg acg cgg gcc gac aag ggg aat gtg ata gaa tgg agc ggg aca      206
Lys Thr Thr Arg Ala Asp Lys Gly Asn Val Ile Glu Trp Ser Gly Thr
                35                  40                  45

aca gtg cgc caa acg gcg cga agt ctg ggg tgg cct atg cca gcg tcg      254
Thr Val Arg Gln Thr Ala Arg Ser Leu Gly Trp Pro Met Pro Ala Ser
            50                  55                  60

aaa ctc gtg ttg att gat gga aat agc atc ttg tat cga gcg ttc ttc      302
Lys Leu Val Leu Ile Asp Gly Asn Ser Ile Leu Tyr Arg Ala Phe Phe
        65                  70                  75

gcg ctg ccg ccg ctc act gcg agg gac ggc acg ccc acg aac gcc gtg      350
Ala Leu Pro Pro Leu Thr Ala Arg Asp Gly Thr Pro Thr Asn Ala Val
    80                  85                  90

tac ggc ttt acg acg atg atc ttg cgg ctg atg tcg gac gaa aag ccg      398
Tyr Gly Phe Thr Thr Met Ile Leu Arg Leu Met Ser Asp Glu Lys Pro
95                  100                 105                 110

acg cat ctg gcc gtc gcg ttt gat aaa tcg aag acg acg ttt cgc cat      446
Thr His Leu Ala Val Ala Phe Asp Lys Ser Lys Thr Thr Phe Arg His
                115                 120                 125

gcg gat ttc gcg gcg tac aaa ggc acg cgc cag gag acg ccc gac gag      494
Ala Asp Phe Ala Ala Tyr Lys Gly Thr Arg Gln Glu Thr Pro Asp Glu
            130                 135                 140

ctc gtg caa cag ttt ccg ctc gcg cgc cgc acc ctg gag gcg ctg tcc      542
Leu Val Gln Gln Phe Pro Leu Ala Arg Arg Thr Leu Glu Ala Leu Ser
        145                 150                 155

atc ccg atg gtc gag atc gac caa tac gag gcc gac gac gtg atc gga      590
Ile Pro Met Val Glu Ile Asp Gln Tyr Glu Ala Asp Asp Val Ile Gly
    160                 165                 170

acg ctc gcc aag cga gcg gcg gag gct ggc ttt gac gtc cgc gtg gtc      638
Thr Leu Ala Lys Arg Ala Ala Glu Ala Gly Phe Asp Val Arg Val Val
175                 180                 185                 190

tcg ggt gac aag gac ctg ttg cag ctg gtc gac gat cgg atc cac gtg      686
Ser Gly Asp Lys Asp Leu Leu Gln Leu Val Asp Asp Arg Ile His Val
                195                 200                 205

ctc ctg acg cgc aag ggc atc acc gag atg gag cac ttt gac gag cag      734
Leu Leu Thr Arg Lys Gly Ile Thr Glu Met Glu His Phe Asp Glu Gln
            210                 215                 220

gcc gtt gcc cgc cgg tat ccg ggg ctc aag ccc gcg cag gtg atc gat      782
Ala Val Ala Arg Arg Tyr Pro Gly Leu Lys Pro Ala Gln Val Ile Asp
        225                 230                 235

ctc aag ggc ctc atg ggc gat ccg tcc gac aac att ccg ggc gtg cct 830
Leu Lys Gly Leu Met Gly Asp Pro Ser Asp Asn Ile Pro Gly Val Pro
    240                 245                 250

ggg gtg ggc gaa aag acg gcg ctc aaa ctg ctc gcg tcc ttt ggc acg 878
Gly Val Gly Glu Lys Thr Ala Leu Lys Leu Leu Ala Ser Phe Gly Thr
255                 260                 265                 270

gtc gag ggc gtg tac gat cac ctc gac gaa gtc cag ggc cag aag ctg 926
Val Glu Gly Val Tyr Asp His Leu Asp Glu Val Gln Gly Gln Lys Leu
                275                 280                 285

cgg gag cgc ctc gag cag cac cgg gag gac gcg ttt ctc tcc aag cgc 974
Arg Glu Arg Leu Glu Gln His Arg Glu Asp Ala Phe Leu Ser Lys Arg
            290                 295                 300

ctc gcc acc atc gcg tgc gac gcg ccc atc gag gtg gat ctc gag acg 1022
Leu Ala Thr Ile Ala Cys Asp Ala Pro Ile Glu Val Asp Leu Glu Thr
        305                 310                 315

ctg cga tac gaa ggg ccg gat ccc gcc cgc gcc atc gcc tgg ttc cgc 1070
Leu Arg Tyr Glu Gly Pro Asp Pro Ala Arg Ala Ile Ala Trp Phe Arg
    320                 325                 330

gag ctc gac ttc cga tcc ctc gtc gac aaa att tcc gag gaa atg agc 1118
Glu Leu Asp Phe Arg Ser Leu Val Asp Lys Ile Ser Glu Glu Met Ser
335                 340                 345                 350

cac gat tcg acg ccg acc cct tcg ccg gcg gcc gcg agc ggc gcg tcg 1166
His Asp Ser Thr Pro Thr Pro Ser Pro Ala Ala Ala Ser Gly Ala Ser
                355                 360                 365

agc gag tgg agt tcc ttt gcg tac ggc ctg att gag gac gcg ggc gcc 1214
Ser Glu Trp Ser Ser Phe Ala Tyr Gly Leu Ile Glu Asp Ala Gly Ala
            370                 375                 380

tgg cag gag gcc atc tcg tcg ttc agc gaa ccg gtc ggc gtc atg atg 1262
Trp Gln Glu Ala Ile Ser Ser Phe Ser Glu Pro Val Gly Val Met Met
        385                 390                 395

gac ttg gcg gat ccg gac tat cac cgg gcg gag atc cgc ggc atg gcc 1310
Asp Leu Ala Asp Pro Asp Tyr His Arg Ala Glu Ile Arg Gly Met Ala
    400                 405                 410

gtc gcg acg ccg aag cgc gcg tac tac gtt cgc ttc ggc gag cga ctg 1358
Val Ala Thr Pro Lys Arg Ala Tyr Tyr Val Arg Phe Gly Glu Arg Leu
415                 420                 425                 430

gag ttg agc gac gtc cgg cca tgg ctc gtg tcg gat cgg ccg aag gtg 1406
Glu Leu Ser Asp Val Arg Pro Trp Leu Val Ser Asp Arg Pro Lys Val
                435                 440                 445

gcg ttc gac ctg aag tcg atg gcg ttc gcg ctg gac gcg cac ggc atc 1454
Ala Phe Asp Leu Lys Ser Met Ala Phe Ala Leu Asp Ala His Gly Ile
            450                 455                 460

gga ttg acg tcg gag tgc ggc tgg cag gac gtg aag ctg gct gcg tac 1502
Gly Leu Thr Ser Glu Cys Gly Trp Gln Asp Val Lys Leu Ala Ala Tyr
        465                 470                 475

ctg ctc aac ccg cag gac ggc gag gtc gaa ctg tcc gat gtg ttc gcc 1550
Leu Leu Asn Pro Gln Asp Gly Glu Val Glu Leu Ser Asp Val Phe Ala
    480                 485                 490

cgc gag cgc ggc cag gaa ctg ccc gcc tgg gag gag ggc gag cgg gag 1598
Arg Glu Arg Gly Gln Glu Leu Pro Ala Trp Glu Glu Gly Glu Arg Glu
495                 500                 505                 510

aag tgg ctc gcc tac acg gcg tct cag ctg cct ccg ctc ttc gag tcc 1646
Lys Trp Leu Ala Tyr Thr Ala Ser Gln Leu Pro Pro Leu Phe Glu Ser

                515                 520                 525

ctg gcg tac acg att cgc atg cag gag atg gag cgg ctg tac caa gag 1694
Leu Ala Tyr Thr Ile Arg Met Gln Glu Met Glu Arg Leu Tyr Gln Glu
            530                 535                 540

gtg gag ctt cct ctg gcg ttc gtg ctg gcg aag atg gag atc acg ggg 1742
Val Glu Leu Pro Leu Ala Phe Val Leu Ala Lys Met Glu Ile Thr Gly
        545                 550                 555

ttt tac gtg aat cgc gag aag ctg gtc gca ttt ggg cag gaa ttg acg 1790
Phe Tyr Val Asn Arg Glu Lys Leu Val Ala Phe Gly Gln Glu Leu Thr
    560                 565                 570

gag cga atc aag cga atc acg cag gag atc tac gat ctc gcg ggc act 1838
Glu Arg Ile Lys Arg Ile Thr Gln Glu Ile Tyr Asp Leu Ala Gly Thr
575                 580                 585                 590

tcg ttc aac ctc aac tcg ccg aag cag ctc ggc gag atc ctg ttc gac 1886
Ser Phe Asn Leu Asn Ser Pro Lys Gln Leu Gly Glu Ile Leu Phe Asp
                595                 600                 605

aag ctc ggc ttg cct gcg ctg aag aag acg aag acc ggc tat tcc acg 1934
Lys Leu Gly Leu Pro Ala Leu Lys Lys Thr Lys Thr Gly Tyr Ser Thr
            610                 615                 620

agc gct gac gtg ctc gaa aag ctg gcg ccc atg cac gag atc gtg cag 1982
Ser Ala Asp Val Leu Glu Lys Leu Ala Pro Met His Glu Ile Val Gln
        625                 630                 635

aag atc ctc gat tat cgt ctg ctg gcg aag ctg cag tcg acg tac gtc 2030
Lys Ile Leu Asp Tyr Arg Leu Leu Ala Lys Leu Gln Ser Thr Tyr Val
    640                 645                 650

gag ggc cta ctg aaa gtc atc cgc aag gag acc ggc cgg gtg cac acc 2078
Glu Gly Leu Leu Lys Val Ile Arg Lys Glu Thr Gly Arg Val His Thr
655                 660                 665                 670

cgg ttt cat cag acg ctc acg gcg acc ggc agg ctc tca agc agc gag 2126
Arg Phe His Gln Thr Leu Thr Ala Thr Gly Arg Leu Ser Ser Ser Glu
                675                 680                 685

ccc aat ctg cag aat atc ccc att cgg ctc gag gag ggg cgg agg ctg 2174
Pro Asn Leu Gln Asn Ile Pro Ile Arg Leu Glu Glu Gly Arg Arg Leu
            690                 695                 700

cgc cag gtg ttc gag ccg acc tac aag gac tgg gtg att ttc gcc gcc 2222
Arg Gln Val Phe Glu Pro Thr Tyr Lys Asp Trp Val Ile Phe Ala Ala
        705                 710                 715

gac tat tcg cag atc gag ttg cgc atc ctg gcc cac ctg tcg ggc gac 2270
Asp Tyr Ser Gln Ile Glu Leu Arg Ile Leu Ala His Leu Ser Gly Asp
    720                 725                 730

gag gcg ctc atc gac gcg ttc cgc cgc gac atg gac atc cac acg cgg 2318
Glu Ala Leu Ile Asp Ala Phe Arg Arg Asp Met Asp Ile His Thr Arg
735                 740                 745                 750

acg gcg gcg gac gtg ttt gag gtg ccg cct gag cag gtg acg agc ctg 2366
Thr Ala Ala Asp Val Phe Glu Val Pro Pro Glu Gln Val Thr Ser Leu
                755                 760                 765

atg cgc cgc cag gcg aag gcc gtg aac ttc ggc atc gtg tac ggg atc 2414
Met Arg Arg Gln Ala Lys Ala Val Asn Phe Gly Ile Val Tyr Gly Ile
            770                 775                 780

agc gat ttc gga ctc gcg cag aac ttg aac atc ccg cag aaa gag gcg 2462
Ser Asp Phe Gly Leu Ala Gln Asn Leu Asn Ile Pro Gln Lys Glu Ala
        785                 790                 795

aaa cgg ttc atc gag agt tat ttc gag aag ttt ccc ggc gtc aag cga     2510
Lys Arg Phe Ile Glu Ser Tyr Phe Glu Lys Phe Pro Gly Val Lys Arg
    800                 805                 810

tac atg gac gag atc gtc aag cag gcg cgc gag cgc ggc tat gtc acg     2558
Tyr Met Asp Glu Ile Val Lys Gln Ala Arg Glu Arg Gly Tyr Val Thr
815                 820                 825                 830

aca ctg atg aac cga cgg cgg tat ctg ccg gac atc cac agc cgc aat     2606
Thr Leu Met Asn Arg Arg Arg Tyr Leu Pro Asp Ile His Ser Arg Asn
                835                 840                 845

tat cag ctc cga agc ttc gcc gag cgc acc gcg atg aac acg ccc atc     2654
Tyr Gln Leu Arg Ser Phe Ala Glu Arg Thr Ala Met Asn Thr Pro Ile
            850                 855                 860

cag gga agc gcc gcg gat ctg atc aag ctc gcg atg gtg cgg atc gat     2702
Gln Gly Ser Ala Ala Asp Leu Ile Lys Leu Ala Met Val Arg Ile Asp
        865                 870                 875

cgc gcc atg cgc gac gcg cag atg gac gcg cgc atg ttg ctc cag gtg     2750
Arg Ala Met Arg Asp Ala Gln Met Asp Ala Arg Met Leu Leu Gln Val
    880                 885                 890

cac gac gag ctg atc ttt gag tgt ccg aag gac gaa ctg gcc gcg ctc     2798
His Asp Glu Leu Ile Phe Glu Cys Pro Lys Asp Glu Leu Ala Ala Leu
895                 900                 905                 910

gaa gtg ctt gtc cga gac aac atg gaa aac gcc atg acc ttg tct gtg     2846
Glu Val Leu Val Arg Asp Asn Met Glu Asn Ala Met Thr Leu Ser Val
                915                 920                 925

ccg ctc aag gtg gat acc gcc tac ggc ccg acg tgg tac gac gcc aag     2894
Pro Leu Lys Val Asp Thr Ala Tyr Gly Pro Thr Trp Tyr Asp Ala Lys
            930                 935                 940

tga ggggggatgc ggcgtgccgg agttgcccga ggtggaaacg gtccgacgcc     2947

acctcgcgga gcgcatcgag ggcgatgtca ttcgcgacgt ggaagtccgc ctgccgcgca     3007

tcgtgcgcca tccggcgctc aacgtgtttg ccgagcggct gagggagcag ggcattcacc     3067

gcgtgggacg gcgcggcaag tatctgttgt tccaattgga tcaggtgctc ctcgtctctc     3127

acctgcgtat     3137

<210>  23
<211>  942
<212>  PRT
<213>  Alicyclobacillus acidocaldarius

<400>  23

Met Asn Phe Val Asn Ile Ile Ile Asn Ser Leu Lys Cys Val Asn Ser
1               5                   10                  15

Ala Cys Val Pro Leu Gly Val Arg Pro Phe Ser Cys Arg His Lys Thr
            20                  25                  30

Thr Arg Ala Asp Lys Gly Asn Val Ile Glu Trp Ser Gly Thr Thr Val
        35                  40                  45

Arg Gln Thr Ala Arg Ser Leu Gly Trp Pro Met Pro Ala Ser Lys Leu
    50                  55                  60

Val Leu Ile Asp Gly Asn Ser Ile Leu Tyr Arg Ala Phe Phe Ala Leu
65                  70                  75                  80

Pro Pro Leu Thr Ala Arg Asp Gly Thr Pro Thr Asn Ala Val Tyr Gly
                85                  90                  95

Phe Thr Thr Met Ile Leu Arg Leu Met Ser Asp Glu Lys Pro Thr His
            100                 105                 110

Leu Ala Val Ala Phe Asp Lys Ser Lys Thr Thr Phe Arg His Ala Asp
        115                 120                 125

Phe Ala Ala Tyr Lys Gly Thr Arg Gln Glu Thr Pro Asp Glu Leu Val
    130                 135                 140

Gln Gln Phe Pro Leu Ala Arg Arg Thr Leu Glu Ala Leu Ser Ile Pro
145                 150                 155                 160

Met Val Glu Ile Asp Gln Tyr Glu Ala Asp Asp Val Ile Gly Thr Leu
                165                 170                 175

Ala Lys Arg Ala Ala Glu Ala Gly Phe Asp Val Arg Val Val Ser Gly
            180                 185                 190

Asp Lys Asp Leu Leu Gln Leu Val Asp Asp Arg Ile His Val Leu Leu
        195                 200                 205

Thr Arg Lys Gly Ile Thr Glu Met Glu His Phe Asp Glu Gln Ala Val
    210                 215                 220

Ala Arg Arg Tyr Pro Gly Leu Lys Pro Ala Gln Val Ile Asp Leu Lys
225                 230                 235                 240

Gly Leu Met Gly Asp Pro Ser Asp Asn Ile Pro Gly Val Pro Gly Val
                245                 250                 255

Gly Glu Lys Thr Ala Leu Lys Leu Leu Ala Ser Phe Gly Thr Val Glu
            260                 265                 270

Gly Val Tyr Asp His Leu Asp Glu Val Gln Gly Gln Lys Leu Arg Glu
        275                 280                 285

Arg Leu Glu Gln His Arg Glu Asp Ala Phe Leu Ser Lys Arg Leu Ala
    290                 295                 300

Thr Ile Ala Cys Asp Ala Pro Ile Glu Val Asp Leu Glu Thr Leu Arg
305                 310                 315                 320

Tyr Glu Gly Pro Asp Pro Ala Arg Ala Ile Ala Trp Phe Arg Glu Leu
                325                 330                 335

Asp Phe Arg Ser Leu Val Asp Lys Ile Ser Glu Glu Met Ser His Asp
            340                 345                 350

Ser Thr Pro Thr Pro Ser Pro Ala Ala Ala Ser Gly Ala Ser Ser Glu
        355                 360                 365

Trp Ser Ser Phe Ala Tyr Gly Leu Ile Glu Asp Ala Gly Ala Trp Gln
    370                 375                 380

Glu Ala Ile Ser Ser Phe Ser Glu Pro Val Gly Val Met Met Asp Leu
385                 390                 395                 400

Ala Asp Pro Asp Tyr His Arg Ala Glu Ile Arg Gly Met Ala Val Ala
                405                 410                 415

Thr Pro Lys Arg Ala Tyr Tyr Val Arg Phe Gly Glu Arg Leu Glu Leu
            420                 425                 430

Ser Asp Val Arg Pro Trp Leu Val Ser Asp Arg Pro Lys Val Ala Phe
        435                 440                 445

Asp Leu Lys Ser Met Ala Phe Ala Leu Asp Ala His Gly Ile Gly Leu
    450                 455                 460

Thr Ser Glu Cys Gly Trp Gln Asp Val Lys Leu Ala Ala Tyr Leu Leu
465                 470                 475                 480

Asn Pro Gln Asp Gly Glu Val Glu Leu Ser Asp Val Phe Ala Arg Glu
                485                 490                 495

Arg Gly Gln Glu Leu Pro Ala Trp Glu Glu Gly Glu Arg Glu Lys Trp
            500                 505                 510

Leu Ala Tyr Thr Ala Ser Gln Leu Pro Pro Leu Phe Glu Ser Leu Ala
        515                 520                 525

Tyr Thr Ile Arg Met Gln Glu Met Glu Arg Leu Tyr Gln Glu Val Glu
    530                 535                 540

Leu Pro Leu Ala Phe Val Leu Ala Lys Met Glu Ile Thr Gly Phe Tyr
545                 550                 555                 560

Val Asn Arg Glu Lys Leu Val Ala Phe Gly Gln Glu Leu Thr Glu Arg
                565                 570                 575

Ile Lys Arg Ile Thr Gln Glu Ile Tyr Asp Leu Ala Gly Thr Ser Phe
            580                 585                 590

Asn Leu Asn Ser Pro Lys Gln Leu Gly Glu Ile Leu Phe Asp Lys Leu
        595                 600                 605

Gly Leu Pro Ala Leu Lys Lys Thr Lys Thr Gly Tyr Ser Thr Ser Ala
    610                 615                 620

Asp Val Leu Glu Lys Leu Ala Pro Met His Glu Ile Val Gln Lys Ile
625                 630                 635                 640

Leu Asp Tyr Arg Leu Leu Ala Lys Leu Gln Ser Thr Tyr Val Glu Gly
                645                 650                 655

Leu Leu Lys Val Ile Arg Lys Glu Thr Gly Arg Val His Thr Arg Phe
            660                 665                 670

His Gln Thr Leu Thr Ala Thr Gly Arg Leu Ser Ser Ser Glu Pro Asn
        675                 680                 685

Leu Gln Asn Ile Pro Ile Arg Leu Glu Glu Gly Arg Arg Leu Arg Gln
    690                 695                 700

Val Phe Glu Pro Thr Tyr Lys Asp Trp Val Ile Phe Ala Ala Asp Tyr
705                 710                 715                 720

Ser Gln Ile Glu Leu Arg Ile Leu Ala His Leu Ser Gly Asp Glu Ala
                725                 730                 735

Leu Ile Asp Ala Phe Arg Arg Asp Met Asp Ile His Thr Arg Thr Ala
            740                 745                 750

Ala Asp Val Phe Glu Val Pro Pro Glu Gln Val Thr Ser Leu Met Arg
        755                 760                 765

Arg Gln Ala Lys Ala Val Asn Phe Gly Ile Val Tyr Gly Ile Ser Asp
    770                 775                 780

Phe Gly Leu Ala Gln Asn Leu Asn Ile Pro Gln Lys Glu Ala Lys Arg
785                 790                 795                 800

Phe Ile Glu Ser Tyr Phe Glu Lys Phe Pro Gly Val Lys Arg Tyr Met
                805                 810                 815

Asp Glu Ile Val Lys Gln Ala Arg Glu Arg Gly Tyr Val Thr Thr Leu
            820                 825                 830

Met Asn Arg Arg Arg Tyr Leu Pro Asp Ile His Ser Arg Asn Tyr Gln
        835                 840                 845

Leu Arg Ser Phe Ala Glu Arg Thr Ala Met Asn Thr Pro Ile Gln Gly
    850                 855                 860

Ser Ala Ala Asp Leu Ile Lys Leu Ala Met Val Arg Ile Asp Arg Ala
865                 870                 875                 880

Met Arg Asp Ala Gln Met Asp Ala Arg Met Leu Leu Gln Val His Asp
                885                 890                 895

Glu Leu Ile Phe Glu Cys Pro Lys Asp Glu Leu Ala Ala Leu Glu Val
            900                 905                 910

Leu Val Arg Asp Asn Met Glu Asn Ala Met Thr Leu Ser Val Pro Leu
        915                 920                 925

Lys Val Asp Thr Ala Tyr Gly Pro Thr Trp Tyr Asp Ala Lys
    930                 935                 940

<210>  24
<211>  1830
<212>  DNA
<213>  Alicyclobacillus acidocaldarius

<220>
<221>  CDS
<222>  (1)..(1830)
<223>

<400>  24
atg gag ctc gac ttc cga tcc ctc gtc gac aaa att tcc gag gaa atg        48
Met Glu Leu Asp Phe Arg Ser Leu Val Asp Lys Ile Ser Glu Glu Met
1               5                   10                  15

agc cac gat tcg acg ccg acc cct tcg ccg gcg gcc gcg agc ggc gcg        96
Ser His Asp Ser Thr Pro Thr Pro Ser Pro Ala Ala Ala Ser Gly Ala
            20                  25                  30

tcg agc gag tgg agt tcc ttt gcg tac ggc ctg att gag gac gcg ggc       144
Ser Ser Glu Trp Ser Ser Phe Ala Tyr Gly Leu Ile Glu Asp Ala Gly
        35                  40                  45

gcc tgg cag gag gcc atc tcg tcg ttc agc gaa ccg gtc ggc gtc atg       192
Ala Trp Gln Glu Ala Ile Ser Ser Phe Ser Glu Pro Val Gly Val Met
    50                  55                  60

atg gac ttg gcg gat ccg gac tat cac cgg gcg gag atc cgc ggc atg       240
Met Asp Leu Ala Asp Pro Asp Tyr His Arg Ala Glu Ile Arg Gly Met
65                  70                  75                  80

gcc gtc gcg acg ccg aag cgc gcg tac tac gtt cgc ttc ggc gag cga       288
Ala Val Ala Thr Pro Lys Arg Ala Tyr Tyr Val Arg Phe Gly Glu Arg
                85                  90                  95

ctg gag ttg agc gac gtc cgg cca tgg ctc gtg tcg gat cgg ccg aag       336
Leu Glu Leu Ser Asp Val Arg Pro Trp Leu Val Ser Asp Arg Pro Lys
            100                 105                 110

gtg gcg ttc gac ctg aag tcg atg gcg ttc gcg ctg gac gcg cac ggc       384
Val Ala Phe Asp Leu Lys Ser Met Ala Phe Ala Leu Asp Ala His Gly
        115                 120                 125

atc gga ttg acg tcg gag tgc ggc tgg cag gac gtg aag ctg gct gcg       432
Ile Gly Leu Thr Ser Glu Cys Gly Trp Gln Asp Val Lys Leu Ala Ala
    130                 135                 140

tac ctg ctc aac ccg cag gac ggc gag gtc gaa ctg tcc gat gtg ttc       480
Tyr Leu Leu Asn Pro Gln Asp Gly Glu Val Glu Leu Ser Asp Val Phe
145                 150                 155                 160

gcc cgc gag cgc ggc cag gaa ctg ccc gcc tgg gag gag ggc gag cgg       528
Ala Arg Glu Arg Gly Gln Glu Leu Pro Ala Trp Glu Glu Gly Glu Arg
                165                 170                 175

gag aag tgg ctc gcc tac acg gcg tct cag ctg cct ccg ctc ttc gag       576
Glu Lys Trp Leu Ala Tyr Thr Ala Ser Gln Leu Pro Pro Leu Phe Glu
            180                 185                 190

tcc ctg gcg tac acg att cgc atg cag gag atg gag cgg ctg tac caa       624

Ser Leu Ala Tyr Thr Ile Arg Met Gln Glu Met Glu Arg Leu Tyr Gln
        195                 200                 205

gag gtg gag ctt cct ctg gcg ttc gtg ctg gcg aag atg gag atc acg      672
Glu Val Glu Leu Pro Leu Ala Phe Val Leu Ala Lys Met Glu Ile Thr
    210                 215                 220

ggg ttt tac gtg aat cgc gag aag ctg gtc gca ttt ggg cag gaa ttg      720
Gly Phe Tyr Val Asn Arg Glu Lys Leu Val Ala Phe Gly Gln Glu Leu
225                 230                 235                 240

acg gag cga atc aag cga atc acg cag gag atc tac gat ctc gcg ggc      768
Thr Glu Arg Ile Lys Arg Ile Thr Gln Glu Ile Tyr Asp Leu Ala Gly
                245                 250                 255

act tcg ttc aac ctc aac tcg ccg aag cag ctc ggc gag atc ctg ttc      816
Thr Ser Phe Asn Leu Asn Ser Pro Lys Gln Leu Gly Glu Ile Leu Phe
            260                 265                 270

gac aag ctc ggc ttg cct gcg ctg aag aag acg aag acc ggc tat tcc      864
Asp Lys Leu Gly Leu Pro Ala Leu Lys Lys Thr Lys Thr Gly Tyr Ser
        275                 280                 285

acg agc gct gac gtg ctc gaa aag ctg gcg ccc atg cac gag atc gtg      912
Thr Ser Ala Asp Val Leu Glu Lys Leu Ala Pro Met His Glu Ile Val
    290                 295                 300

cag aag atc ctc gat tat cgt ctg ctg gcg aag ctg cag tcg acg tac      960
Gln Lys Ile Leu Asp Tyr Arg Leu Leu Ala Lys Leu Gln Ser Thr Tyr
305                 310                 315                 320

gtc gag ggc cta ctg aaa gtc atc cgc aag gag acc ggc cgg gtg cac     1008
Val Glu Gly Leu Leu Lys Val Ile Arg Lys Glu Thr Gly Arg Val His
                325                 330                 335

acc cgg ttt cat cag acg ctc acg gcg acc ggc agg ctc tca agc agc     1056
Thr Arg Phe His Gln Thr Leu Thr Ala Thr Gly Arg Leu Ser Ser Ser
            340                 345                 350

gag ccc aat ctg cag aat atc ccc att cgg ctc gag gag ggg cgg agg     1104
Glu Pro Asn Leu Gln Asn Ile Pro Ile Arg Leu Glu Glu Gly Arg Arg
        355                 360                 365

ctg cgc cag gtg ttc gag ccg acc tac aag gac tgg gtg att ttc gcc     1152
Leu Arg Gln Val Phe Glu Pro Thr Tyr Lys Asp Trp Val Ile Phe Ala
    370                 375                 380

gcc gac tat tcg cag atc gag ttg cgc atc ctg gcc cac ctg tcg ggc     1200
Ala Asp Tyr Ser Gln Ile Glu Leu Arg Ile Leu Ala His Leu Ser Gly
385                 390                 395                 400

gac gag gcg ctc atc gac gcg ttc cgc cgc gac atg gac atc cac acg     1248
Asp Glu Ala Leu Ile Asp Ala Phe Arg Arg Asp Met Asp Ile His Thr
                405                 410                 415

cgg acg gcg gcg gac gtg ttt gag gtg ccg cct gag cag gtg acg agc     1296
Arg Thr Ala Ala Asp Val Phe Glu Val Pro Pro Glu Gln Val Thr Ser
            420                 425                 430

ctg atg cgc cgc cag gcg aag gcc gtg aac ttc ggc atc gtg tac ggg     1344
Leu Met Arg Arg Gln Ala Lys Ala Val Asn Phe Gly Ile Val Tyr Gly
        435                 440                 445

atc agc gat ttc gga ctc gcg cag aac ttg aac atc ccg cag aaa gag     1392
Ile Ser Asp Phe Gly Leu Ala Gln Asn Leu Asn Ile Pro Gln Lys Glu
    450                 455                 460

gcg aaa cgg ttc atc gag agt tat ttc gag aag ttt ccc ggc gtc aag     1440
Ala Lys Arg Phe Ile Glu Ser Tyr Phe Glu Lys Phe Pro Gly Val Lys
465                 470                 475                 480

cga tac atg gac gag atc gtc aag cag gcg cgc gag cgc ggc tat gtc      1488
Arg Tyr Met Asp Glu Ile Val Lys Gln Ala Arg Glu Arg Gly Tyr Val
                485                 490                 495

acg aca ctg atg aac cga cgg cgg tat ctg ccg gac atc cac agc cgc      1536
Thr Thr Leu Met Asn Arg Arg Arg Tyr Leu Pro Asp Ile His Ser Arg
            500                 505                 510

aat tat cag ctc cga agc ttc gcc gag cgc acc gcg atg aac acg ccc      1584
Asn Tyr Gln Leu Arg Ser Phe Ala Glu Arg Thr Ala Met Asn Thr Pro
        515                 520                 525

atc cag gga agc gcc gcg gat ctg atc aag ctc gcg atg gtg cgg atc      1632
Ile Gln Gly Ser Ala Ala Asp Leu Ile Lys Leu Ala Met Val Arg Ile
    530                 535                 540

gat cgc gcc atg cgc gac gcg cag atg gac gcg cgc atg ttg ctc cag      1680
Asp Arg Ala Met Arg Asp Ala Gln Met Asp Ala Arg Met Leu Leu Gln
545                 550                 555                 560

gtg cac gac gag ctg atc ttt gag tgt ccg aag gac gaa ctg gcc gcg      1728
Val His Asp Glu Leu Ile Phe Glu Cys Pro Lys Asp Glu Leu Ala Ala
                565                 570                 575

ctc gaa gtg ctt gtc cga gac aac atg gaa aac gcc atg acc ttg tct      1776
Leu Glu Val Leu Val Arg Asp Asn Met Glu Asn Ala Met Thr Leu Ser
            580                 585                 590

gtg ccg ctc aag gtg gat acc gcc tac ggc ccg acg tgg tac gac gcc      1824
Val Pro Leu Lys Val Asp Thr Ala Tyr Gly Pro Thr Trp Tyr Asp Ala
        595                 600                 605

aag tga                                                              1830
Lys

<210>  25
<211>  609
<212>  PRT
<213>  Alicyclobacillus acidocaldarius

<400>  25

Met Glu Leu Asp Phe Arg Ser Leu Val Asp Lys Ile Ser Glu Glu Met
1               5                   10                  15

Ser His Asp Ser Thr Pro Thr Pro Ser Pro Ala Ala Ala Ser Gly Ala
            20                  25                  30

Ser Ser Glu Trp Ser Ser Phe Ala Tyr Gly Leu Ile Glu Asp Ala Gly
        35                  40                  45

Ala Trp Gln Glu Ala Ile Ser Ser Phe Ser Glu Pro Val Gly Val Met
    50                  55                  60

Met Asp Leu Ala Asp Pro Asp Tyr His Arg Ala Glu Ile Arg Gly Met
65                  70                  75                  80

Ala Val Ala Thr Pro Lys Arg Ala Tyr Tyr Val Arg Phe Gly Glu Arg
                85                  90                  95

Leu Glu Leu Ser Asp Val Arg Pro Trp Leu Val Ser Asp Arg Pro Lys

            100                 105                 110

Val Ala Phe Asp Leu Lys Ser Met Ala Phe Ala Leu Asp Ala His Gly
        115                 120                 125

Ile Gly Leu Thr Ser Glu Cys Gly Trp Gln Asp Val Lys Leu Ala Ala
    130                 135                 140

Tyr Leu Leu Asn Pro Gln Asp Gly Glu Val Glu Leu Ser Asp Val Phe
145                 150                 155                 160

Ala Arg Glu Arg Gly Gln Glu Leu Pro Ala Trp Glu Glu Gly Glu Arg
                165                 170                 175

Glu Lys Trp Leu Ala Tyr Thr Ala Ser Gln Leu Pro Pro Leu Phe Glu
            180                 185                 190

Ser Leu Ala Tyr Thr Ile Arg Met Gln Glu Met Glu Arg Leu Tyr Gln
        195                 200                 205

Glu Val Glu Leu Pro Leu Ala Phe Val Leu Ala Lys Met Glu Ile Thr
    210                 215                 220

Gly Phe Tyr Val Asn Arg Glu Lys Leu Val Ala Phe Gly Gln Glu Leu
225                 230                 235                 240

Thr Glu Arg Ile Lys Arg Ile Thr Gln Glu Ile Tyr Asp Leu Ala Gly
                245                 250                 255

Thr Ser Phe Asn Leu Asn Ser Pro Lys Gln Leu Gly Glu Ile Leu Phe
            260                 265                 270

Asp Lys Leu Gly Leu Pro Ala Leu Lys Lys Thr Lys Thr Gly Tyr Ser
        275                 280                 285

Thr Ser Ala Asp Val Leu Glu Lys Leu Ala Pro Met His Glu Ile Val
    290                 295                 300

Gln Lys Ile Leu Asp Tyr Arg Leu Leu Ala Lys Leu Gln Ser Thr Tyr
305                 310                 315                 320

Val Glu Gly Leu Leu Lys Val Ile Arg Lys Glu Thr Gly Arg Val His
                325                 330                 335

Thr Arg Phe His Gln Thr Leu Thr Ala Thr Gly Arg Leu Ser Ser Ser
            340                 345                 350

Glu Pro Asn Leu Gln Asn Ile Pro Ile Arg Leu Glu Glu Gly Arg Arg
        355                 360                 365

Leu Arg Gln Val Phe Glu Pro Thr Tyr Lys Asp Trp Val Ile Phe Ala
    370                 375                 380

Ala Asp Tyr Ser Gln Ile Glu Leu Arg Ile Leu Ala His Leu Ser Gly
385                 390                 395                 400

Asp Glu Ala Leu Ile Asp Ala Phe Arg Arg Asp Met Asp Ile His Thr
                405                 410                 415

Arg Thr Ala Ala Asp Val Phe Glu Val Pro Pro Glu Gln Val Thr Ser
            420                 425                 430

Leu Met Arg Arg Gln Ala Lys Ala Val Asn Phe Gly Ile Val Tyr Gly
        435                 440                 445

Ile Ser Asp Phe Gly Leu Ala Gln Asn Leu Asn Ile Pro Gln Lys Glu
    450                 455                 460

Ala Lys Arg Phe Ile Glu Ser Tyr Phe Glu Lys Phe Pro Gly Val Lys
465                 470                 475                 480

Arg Tyr Met Asp Glu Ile Val Lys Gln Ala Arg Glu Arg Gly Tyr Val
                485                 490                 495

Thr Thr Leu Met Asn Arg Arg Arg Tyr Leu Pro Asp Ile His Ser Arg
            500                 505                 510

Asn Tyr Gln Leu Arg Ser Phe Ala Glu Arg Thr Ala Met Asn Thr Pro
        515                 520                 525

Ile Gln Gly Ser Ala Ala Asp Leu Ile Lys Leu Ala Met Val Arg Ile
    530                 535                 540

Asp Arg Ala Met Arg Asp Ala Gln Met Asp Ala Arg Met Leu Leu Gln
545                 550                 555                 560

Val His Asp Glu Leu Ile Phe Glu Cys Pro Lys Asp Glu Leu Ala Ala
                565                 570                 575

Leu Glu Val Leu Val Arg Asp Asn Met Glu Asn Ala Met Thr Leu Ser
            580                 585                 590

Val Pro Leu Lys Val Asp Thr Ala Tyr Gly Pro Thr Trp Tyr Asp Ala
        595                 600                 605

Lys

<210>  26
<211>  30
<212>  DNA
<213>  Artificial

<220>
<223>  ENZO

<400>  26

cgctgcacat ggcctggggc ctcctgctca                                    30

<210>  27
<211>  36
<212>  DNA
<213>  Artificial

<220>
<223>  SMAP

<400>  27
tttatatata tataaacccc tgcactgttt cccaga                              36

<210>  28
<211>  16
<212>  DNA
<213>  Artificial

<220>
<223>  Loop

<400>  28
atccggatgt aggatc                                                    16

<210>  29
<211>  18
<212>  DNA
<213>  Artificial

<220>
<223>  Outer forward

<400>  29
gatggtgacc acctcgac                                                  18

<210>  30
<211>  18
<212>  DNA
<213>  Artificial

<220>
<223>  Outer Reverse

<400>  30
tgtacccttc ctccctcg                                                  18

<210>  31
<211>  30
<212>  DNA
<213>  Artificial

<220>
<223>  primer

<400>  31
cgagtacggg cccacactca cagttttcac                                    30

<210>  32
<211>  32
<212>  DNA
<213>  Artificial

<220>
<223>  primer

<400>  32
tttatatata tataaaccgg gagttgggcg ag                                    32

<210>  33
<211>  15
<212>  DNA
<213>  Artificial

<220>
<223>  primer

<400>  33
gcaggcatac actga                                                       15

<210>  34
<211>  18
<212>  DNA
<213>  Artificial

<220>
<223>  primer

<400>  34
cctgagcccc cagcaggt                                                    18

<210>  35
<211>  18
<212>  DNA
<213>  Artificial

<220>
<223>  primer

<400>  35
acaagatgtc ggggagtg                                                    18

<210>  36
<211>  30
<212>  DNA
<213>  Artificial

<220>
<223>  primer

<400>  36
tactggagac gtgaaattgg gtgaggatgc                                       30

<210>  37
<211>  27
<212>  DNA
<213>  Artificial

<220>
<223>  primer

<400>  37
agaaggaggt gtaccattgc cactgtt                                          27

<210>  38
<211>  17
<212>  DNA
<213>  Artificial

<220>
<223>  primer

<400>  38
cacactggaa ttggggg                                                      17


<210>  39
<211>  18
<212>  DNA
<213>  Artificial

<220>
<223>  primer

<400>  39
tcagctatct tctcctgg                                                     18


<210>  40
<211>  18
<212>  DNA
<213>  Artificial

<220>
<223>  primer

<400>  40
tgtgatattc tcaccttc                                                     18